# EUROPEAN PATENT APPLICATION

(11) **EP 1 857 818 A1**
(43) Date of publication of application: **21.11.2007**
(21) Application number: 06009954.6
(22) Date of filing: 15.05.2006
(51) Int. Cl.: G01N 33/68, C07K 14/765, C07K 14/575, A61P 3/10

(54) **Diagnostic and therapeutic uses of peptides for pre-forms of type 2 diabetes and conditions associated therewith**

(71) Applicant: DIGILAB BioVisioN GmbH, 30173 Hannover (DE)
(72) Inventor: Budde, Petra, Dr., 30655 Hannover (DE); Zucht, Hans-Dieter, Dr., 30539 Hannover (DE); Tammen, Harald, Dr., 30449 Hannover (DE); Hess, Rüdiger, Dr., 30625 Hannover (DE); Menzel, Christoph, Dr, 40724 Hilden (DE)
(74) Representative: Kröncke, Rolf

(57) **Abstract**

The present invention is in the field of diagnostics and therapeutics. Specifically, the present invention provides methods for diagnosis and therapy of diseases and conditions associated with oxidative stress such as Type 2 diabetes, hyperglycaemia, preeclampsia, aging, etc. Furthermore the invention provides peptides, binding agents recognizing said peptides, diagnostic assay kits, and compositions suitable for medical purposes. The peptides of the invention are characterized by a C-terminal, non-enzymatic originated amide or by a nitric oxide binding site. The peptides of the invention are suitable as biomarker or as therapeutic substances capable to function as nitric oxide delivery vehicles.

## Description

### FIELD OF THE INVENTION

The present invention lies in the field of diagnostics and therapeutics. Specifically, the present invention provides methods for diagnosis and therapy of diseases and conditions associated with oxidative stress such as Type 2 diabetes, hyperglycaemia, preeclampsia, aging, and pre-forms of type 2 diabetes such as impaired glucose tolerance (IGT) representing a pre-stage of diabetes, etc. Furthermore the invention provides peptides, binding agents recognizing said peptides, diagnostic assay kits, and compositions suitable for medical purposes. Some of the peptides of the invention are characterized by a C-terminal, non-enzymatic originated amide or by a nitric oxide binding site. On the one hand, the peptides of the invention are suitable as biomarker or as therapeutic substances capable to function as nitric oxide delivery vehicles. On the other hand, the peptides of the invention are suitable as marker molecules for pre-forms of type 2 diabetes and for type 2 diabetes.

### BACKGROUND OF THE INVENTION

Type 2 diabetes, pre-forms of type 2 diabetes such as impaired glucose tolerance (IGT), obesity, metabolic syndrome, cardiovascular diseases and atherosclerosis are all diseases interconnected to each other.

Impaired glucose tolerance or IGT is the name given to define blood glucose levels that are higher than normal, but below the level of a person with diabetes. IGT is a combination of (i) impaired secretion of insulin and (ii) reduced insulin sensitivity (insulin resistance).

Further, IGT is often associated with a cluster of inter-related cardiovascular risk factors known as the Metabolic Syndrome, Insulin Resistance Syndrom or Syndrome X.

Normally the so called oral glucose tolerance test (oGTT) is applied to determine the presence of IGT or diabetes. That is, in people with IGT, the rise in blood glucose that occurs after consuming 75g glucose is greater than normal and especially is more long lasting, i.e more than 6,7 mM and below 10 mM plasma glucose. Diabetic level starts with glucose levels of 10 mM or higher determined two hours after consumption of 75g glucose as defined by the WHO. Fasting blooded glucose levels are normal or moderately raised. IGT carries a high risk of progressing to type 2 diabetes, leading to it being referred to as "pre-diabetes" by e.g. the American Diabetes Association or borderline diabetes or subclinical diabetes. That is, some people who develop IGT may revert to normal glucose tolerance. Others will remain in a state of IGT. However, once IGT has developed, the body's insulin secretion and sensitivity tend to continue to decline, ultimately resulting in type 2 diabetes. Actually, about 40 to 50% of individuals with IGT will develop type 2 diabetes (accompanied by an increased risk of cardiovascular disease and microvascular complications) within ten years. It is estimated that 314 million people had IGT with the South-East Asia Region currently having the highest number of people with IGT and the highest prevalence rate. It was found that the prevalence of IGT increases with age, that there is no significant difference between men and women, that certain ethnic groups have a higher prevalence of IGT and that the prevalence of IGT in Europe is similar to that in the USA.

Another study revealed that people with type 2 diabetes were more than twice as likely to die during the follow-up period than people with normal blood glucose control. People with IGT were 50% more likely to die of cardiovascular complications during follow-up than people with normal blood glucose control.

Today IGT is determined using the oral glucose tolerance test (oGTT) as indicated above, the individuals have to consume 75g glucose and blood glucose level is determined after 1 and 2 hours, respectively. Alternatively the fasting plasma glucose test is applied where people are asked not to eat anything before the sample is taken. People who have IGT are rarely treated because the condition is rarely diagnosed. IGT can be prevented by increased physical activity, maintaining a healthy weight, and following a healthy balanced diet.

In view of the above, there still exist a demanding interest in providing means for allowing the determination and the diagnosis of IGT.

The metabolic syndrome is a cluster of the most dangerous cardiovascular diseases (CVD) risk factors: diabetes or pre-diabetes, abdominal obesity or obesity, changes in cholesterol and hypertension. Abdominal obesity or obesity is mainly responsible for the rising prevalence of metabolic syndrome. Obesity is associated with raised circulating levels of several acute-phase proteins and inflammatory cytokines that contribute to a state of low-grade inflammation that is linked causally to insulin resistance. Insulin resistance is present in the majority of people with the metabolic syndrome and is also strongly associated with cardiovascular diseases risk and Type 2 diabetes.

Adipose tissue or fat cells secrete a diverse range of protein factors that are collectively named "adipokines". These adipokines are involved in lipid metabolism (retinol binding protein, cholesterol ester transfer protein), insulin sensitivity, the alternative complement system, vascular haemostasis (plasminogen activator inhibitor-1; PAI-1), blood pressure regulation (angiotensinogen) and angiogenesis, as well as the regulation of energy balance. In addition, there is a growing list of adipokines involved in inflammation (TNF-alpha, IL-1, IL-6, IL-8, IL-10, transforming growth factor-, nerve growth factor) and acute-phase response (haptoglobin, serum amyloid A) (Acta Physiol Scand, 2005, 184:285-93).

### Diabetes and oxidative stress

Many of the complications related to diabetes are explained by a hyperglycaemia-induced increase of oxidative stress (Cell Biochem Biophys, 2005, 43:289-330, Free Radical Biol Med, 2003, 34:1563-75). Macrophages, which are present in fat tissue to a large extent, can produce large amounts of nitric oxide. Reactive oxidative species (ROS) and reactive nitrogen species (RNS), generated by various pathways, can deplete the antioxidant defense, rendering the effected cells and tissues increasingly vulnerable to oxidative damage. Oxidative damage effects nearly all biological structures such as DNAs, lipids, and proteins, leading to structural and functional alterations of the effected cells and tissues. Furthermore recent studies indicate, that ROS are important second messengers in the regulation of intracellular signalling and therefore may also impair intracellular signalling (Cell Biochem Biophys, 2005, 43:289-330). Cardiovascular risk factors are also associated with an imbalance of the redox equilibrium toward oxidative stress, leading to endothelial activation and proinflammatory processes implicated in atherogenesis and metabolic disorders. This among others leads to cardiovascular diseases such as congestive heart failure and diabetes-associated heart dysfunction (diabetic cardiomyopathy) (Acta Pharmacol Sin, 2005, 26:908-17; Antioxid Redox Signal, 2005, 7-8:1062-70).

Diabetes is primary characterized by insufficient insulin secretion to lower blood glucose levels appearing after food ingestion. Consequently upregulation of the insulin secretion is one of the primary goals in treatment of diabetes. Insulin secretion is promoted by a class of protein termed incretins. Glucose-dependent insulintropic peptide (GIP) and Glucagon-like peptide 1 (GLP-1) are two example of such incretins upregulating insulin secretion. The precursor of GIP having 153 amino acids in size, is produced as a molecule, which comprises a signal sequence, followed by a N-terminal pro-peptide, the active GIP-peptide and finally a C-terminal pro-peptide (Seq-ID. Nos 18 to 23). The peptide is produced in the small bowel, it is released into the blood stream and goes to the pancreas. Secretion of GIP follows nutrient ingestion. The primary action of the mature GIP corresponding to amino acids 52 to 93 is the stimulation of glucose-dependent insulin secretion. GIP may also play a role in adipocyte biology. GIP may also play a role in obesity and obesity-related hyperglycemia and dyslipidemia since the GIP receptor is expressed on adipocytes

To generate active GIP the signal sequence, and the N- and the C-terminal pro-peptides need to be removed from the precursor of GIP resulting in release of the N-terminal pro-peptide (in the following also referred to as N-terminal proGIP), the C-terminal pro-peptide (in the following also referred to as C-terminal proGIP) and the active GIP. The N-terminal two amino acids residues of active GIP are rapidly removed by Dipeptidyl Peptidase 4 (DDP4) resulting in inactivation of active GIP to inactive GIP and subsequently in rapid degradation of the remainder of the inactive GIP. Consequently GIP has a very short half life in vivo, which is only about 2 minutes and is difficult to measure. The half-lives and concentrations of the N-or the C-terminal propeptides of GIP are unknown, as there do not exist any methods to measure these peptides. As the baseline concentration of GIP is very low, a stimulus, such as a glucose stimulus, for example, in the context of an oral glucose tolerance test, is needed to elevate the low baseline levels of GIP to concentrations within the sensitivity ranges of the GIP assays available. In addition it is necessary to protect the low levels of active GIP present in the samples from rapid degradation by DPP4, as DPP4 is also present in plasma, by adding DPP4 inhibitors into the blood samples directly after drawing the blood from the individual or patient to be tested. These hurdles result in that the determination of GIP in plasma is very difficult. Studies measuring the plasma concentration of GIP in normal individuals and in individuals suffering from impaired glucose tolerance (IGT), a pre-form of diabetes, come to contradictory results with some showing that IGT-patients show increased GIP plasma levels and other studies showing that IGT-patients show decreased GIP plasma levels, as compared to healthy controls (Metabolics, 2004, 53:624, Diabeters Care, 2004, 27:1696).

Today, for the diagnosis of IGT, the amount of the GIP in its active or inactive form is determined after applying e.g an oGTT. However, typically the determination of both the active and inactive form of GIP does not allow drawing any conclusion regarding the presence of IGT or diabetes since the ratio of active vs. inactive GIP depends in the acitivity and amount of the DPP-4 rather than representing a surrogate marker for the amount of insulin. In addition, both peptides are degraded rapidly.

As DPP4 indirectly lowers insulin secretion by inactivation of the insulin secretion-promoting GIP, DPP4-inhibitors are potential drugs to treat diseases causes or associated with too low insulin secretion, such as diabetes and related diseases and disorders. However insulin secretion is not only dependent or regulated by GIP. Other factors also influence insulin secretion and may in combination with insufficient GIP or completely independent of GIP be responsible for insufficient insulin secretion in diabetes. This may vary from patient to patient and therefore diabetes patients may be tested for GIP concentrations to determine whether treatment with DPP4-inhibitors makes sense for the individual patient or not.

The problem to be solved is to provide an assay to measure GIP,
- which assay is not dependent on stimulation, for example by oGTT, of the baseline GIP concentration to obtain levels of GIP within the measurement range of the assay,
- which assay is not dependent on addition of DPP4-inhibitors to the sample to prevent degradation of GIP before or during the assay,
- which assay is not influenced by the very short in vivo and in vitro half-live of GIP, and
- which assay also depicts to some extent the overall GIP concentration over a period of time (similar to C-peptide assays depicting to some extent the long-term concentration of insulin in for example diabetic patients).

Human serum albumin is the most abundant protein in human blood plasma. It is produced in the liver. The reference range for albumin concentrations in blood is 30 to 50 g/L (3.0 to 5.0 g/dL). It has a serum half-life of approximately 20 days and a molecular mass of 67 kDa.

At the N-terminus human serum albumin comprises the amino acid sequence Asp-Ala-His-Lys (DAHK), which represents a high affinity binding site for cationic peptides or metal ions such as nickel, cobalt and copper ions. For example, in cardiac ischemia, stroke etc. albumin comprises an unknown kind of modification, which highly impairs this high affinity binding site. Consequently a decrease of this binding activity of albumin for metal ions is used in a clinical approved Ischemia Modified Albumin (IMA)-assay to diagnose ischemia. The IMA-assay for ischemia measures a transitory (6 to 24 hours) decline in cobalt (II) binding to human albumin in serum sample (Clinical Chemistry, 2004, 50:1063-5).

In addition to its metal ion binding site albumin comprises a binding site for nitric oxide (NO). This binding site represents a free sulfhydryl group present in a cystein side chain, which side chain is not involved in formation of a disulfide bridge within the albumin molecule. Albumin with NO bound to this free sulfhydryl group is termed S-nitroso-albumin. S-nitroso-albumin is thought to be an important in vivo NO-donor, and NO has some biological very important activities such as regulation of the vascular tonus and blood pressure, functions in neuronal transmission and inflammation. S-nitroso-albumin, as a reservoir of nitric oxide, is proposed to be an important vascular tonus regulator which slowly releases NO, resulting in continuous lowering of blood pressure (Biochem Biophys Res Commun, 1996, 225:112-5). As a consequence inhaled nitric oxide gas is used as a selective pulmonary vasodilator and is meanwhile an important therapeutic agent, for example in treatment of postoperative pulmonary hypertension.

US 6,620,786 B2 claims a biopolymer marker peptide consisting of amino acid residues 25 to 50 of human albumin as a diagnostic marker for insulin resistance. However in US 6,620,786 B2 only a non-modified albumin sequence is described and claimed.

Surprisingly the present inventors found, that a distinct, previously unknown modification of this albumin peptide, namely a C-terminal amidated peptide, may be suitable as a biomarker for diabetes and related diseases and conditions. This is even more surprising, as this amidated peptide cannot be originated from enzymatic activity of the only enzyme known to catalyze C-terminal peptide amidations, the peptidylglycine alpha-amidating monooxygenase. This peptidylglycine alpha-amidating monooxygenase requires for it's activity a Glycine amino acid residue at the very next position of the C-terminal side following the amino acid residue to be amidated, The peptidylglycine alpha-amidating monooxygenase-enzyme cannot amidate the C-terminus of the human albumin peptide representing amino acid residues 25 to 51 and also cannot amidate almost all fragments of the human albumin peptide representing amino acid residues 25 to 51. The peptidylglycine alpha-amidating monooxygenase cleaves the peptide bond between two amino acid residues having a relative position of 0 and -1 and in the process of this reaction the C-termial amino acid residue (position 0) of one of the cleavage products is amidated.

Not to be bound by theory, we propose a mechanism, which explains the occurrence of this fragment of human albumin comprising an unusual C-terminal amide-modification. We propose that this peptide-amide originated as a consequence of a non-enzymatic peptide alpha-amidation. A non-enzymatic peptide alpha-amidation has been described in the prior art involving the Fenton reaction (J Biol Chem, 1985, 260:9088-91).
- Copper ions (Cu2+) catalyze the reduction of molecular oxygen (02) to hydrogen peroxide (H2O2) in the presence of redicing agents or antioxidants, for example ascorbate.
- Substances such as ascorbate can reduce Cu2+ ions, which are coordinated to peptides or proteins to Cu1+ ions.
- Reduced copper ions (Cu1+), coordinated to peptides or proteins react with hydrogen peroxide (H2O2), producing hydroxyl ions (OH1-) and hydroxyl radicals (*OH) (= Fenton reaction)
- Hydroxyl radicals (*OH) react with a sterically near-by peptide chain (in the case of albumin for example the of amino acid no. 51) which finally results in cleavage of the peptide bond (in the case of albumin for example between amino acid 52 and 51). The resulting peptides posses either at their C-terminus an amide group or at their N-terminus a carbonyl group.

The described mechanism tries to explain theoretically the occurrence of the C-terminal alpha-amide in the albumin fragments identified in plasma of healthy humans subjected to an oral glucose tolerance test. However the actual mechanism might be different. For the mechanism proposed the peptide bond cleaved must not necessarily be the bond between amino acid 52 and 51 of albumin. Other peptide bonds, which are sterically close to the copper-binding site within the 3D-structure of serum albumin, should also be susceptible to this reaction. Consequently we not only identified the albumin fragments representing amino acids 25-51 with a C-terminal alpha-amide group, but in addition also the albumin fragments representing amino acids 25-50, 25-48, 25-47 and 25-37.

It is expected that further albumin fragments with an C-terminal alpha-amide group such as albumin 25-57, 25-56, 25-55, 25-54, 25-53, 25-52, 25-49, 25-46, 25-45, 25-44, 25-43, 25-42, 25-41, 25-40, 25-39, 25-38, 25-36, 25-35, 25-34, 25-33, 25-32, 25-31, 25-30, 25-29, 25-28, 25-27 and albumin 25-26 are also present in vivo and have similar characteristics as the peptides identified by us. Besides the N-terminal fragments listed above the corresponding remaining second fragments of albumin are also of interest and are also potentially present in vivo.

These second fragments are characterized by having an N-terminal modification of a carbonyl group.

In addition to the copper binding site present in the albumin molecule close to it's cleavage site resulting in C-terminal alpha-amidated peptides, there is another potentially relevant structural feature closely located to said cleavage site, namely a cystein with a free sulfhydryl group. This sulfhydryl group is known to bind for example nitric oxide. Nitric oxide could also be involved in the mechanism of cleavage.

These novel albumin C-terminal alpha-amidated peptides might be useful as a biomarker or therapeutic for diseases or conditions like IGT associated with oxidative stress. The fragment of albumin, remaining after release of said novel albumin C-terminal alpha-amidated peptides, might also be useful as a biomarker or therapeutic, for example as an NO scavenger or, if NO is bound, as an NO donor. NO is important in several biological systems including in regulation of vasopermeability and vasoconstriction.

In addition, the invention solves the problem by providing an assay to measure pro-peptides of GIP, which are generated during maturation of active GIP. These pro-peptides have never been measured and their half-live is unknown to date. Surprisingly it is possible to measure these pro-peptides as a surrogate for GIP in plasma of healthy individuals and in plasma of individuals suffering from impaired glucose tolerance (IGT), a pre-form of diabetes. The results of the invention reveal for the first time, that these pro-peptides are measurable and that these pro-peptides are elevated in plasma of IGT patient and in plasma of normal, healthy individuals, if a glucose stimulus is applied. Addition of DPP4-inhibitors to the plasma samples is not necessary for measuring these pro-peptides of GIP in plasma. The fact that it was possible to measure the pro-peptides of GIP was surprising, as the concentration of the mature GIP, during whose maturation these pro-peptides are generated from the GIP-precursor, is very low and as these pro-peptides were unknown to exist or to be stable in the circulation.

It is assumed that the pro-peptides of GIP may have a long in vivo (and in vitro) half-life, as compared to GIP, as they are measurable in plasma and as they are not subject to DPP4 degradation. In addition there is not known any function for these pro-peptides and consequently there is no biological need to strictly limit their half-life in vivo. In addition and surprisingly the C-terminal amino acid residue arginine in the pro-peptides of GIP we identified, namely GIP (22-51) and GIP (30-51) is not removed by endoproteinases, as would be expected.

### SUMMARY OF THE INVENTION

In a first aspect the present invention provides a method for diagnosing pre-forms of tpe 2 diabetes, such as IGT, in an individual comprising the steps of:
a. determining the relative or absolute amount of a at least one peptide selected from the group consisting of (i) N-terminal or C-terminal proGIP having the Seq-ID No.19 to 21 or a modified form thereof or (ii) at least one peptide comprising a C-terminal alpha-amidation, said amidated peptide is derived from a precursor peptide or protein and at least the C-terminal amino acid of said precursor peptide or protein is absent in said amidated peptide, whereby the alpha-amidation can not be originated from enzymatic cleavage and amidation in a sample of an individual;
b. comparing the result of a) with the result determined using a negative control sample or with an already known reference value; and
   determining the presence or absence of a pre-form of type 2 diabetes in said individual.

In particular determining the amount of C-terminal amidated alpha-amidation of human albumin and/or of N-terminal or C-terminal proGIP, in particular of N-terminal proGIP or modifications of said peptides allows to diagnose the presence of a pre-form of type 2 diabetes, such as IGT, in an individual.

In a second aspect the invention provides a method for detecting oxidative stress in an individual, like a living organism, or a sample thereof by determining the relative or absolute amount of at least one peptide comprising a C-terminal alpha-amidation, said amidated peptide is derived from a precursor peptide or protein and at least the C-terminal amino acid of said precursor peptide or protein is absent in said amidated peptide, whereby the alpha-amidation can not be originated from enzymatic cleavage and amidation, the method comprising the step of measuring the amount of said at least one peptide in an individual or a sample of said individual and comparing the determined amount with a reference amount of said at least one peptide.

Preferably the precursor peptide or protein, from which said peptide originates, comprises (i) a binding site for copper, cobalt, nickel or iron ions, and/or (ii) a free sulfhydryl group, and/or (iii) a nitric oxide binding site.

Preferably the oxidative stress detected with the methods of the invention reflects a condition selected from insulin resistance, glucose intolerance, hyperglycaemia, Type 2 diabetes, ischemia, stroke, hypoxemia, preeclampsia, trauma, coagulation disorders, sepsis, and deprivation of anti oxidants, copper, iron, cobalt or nickel. That is, the degree of oxidative stress of an individual represents an indicator for various diseases and conditions mentioned above.

In a further aspect the invention provides a method for monitoring the therapy of a condition selected from insulin resistance, glucose intolerance, hyperglycaemia, Type 2 diabetes, ischemia, stroke, hypoxemia, preeclampsia, trauma, coagulation disorders, sepsis, and deprivation of anti oxidants, copper, iron, cobalt or nickel or for monitoring of aging, for monitoring of narcosis during surgery, or to detect the use of performance-enhancing substances by an athlete or to detect the application of performance-enhancing substances to animals used in sports by measurement of the oxidative stress.

Preferably the method used to measure oxidative stress, or to detect a said condition or to monitor the therapy of a said condition by determination of said at least one peptide is done by a method selected from the list comprising ELISA, RIA, Western blotting, FACS analysis, plasmon resonance, immuno precipitation, and mass spectrometry. Preferably said at least one peptide detected is a fragment of serum albumin, more preferably said at least one peptide is selected from Seq.-ID 1 to 5.

In a further aspect the invention provides a serum albumin peptide comprising the sequence Asp-Ala-His-Xaa (Seq.-ID 17), wherein Xaa represents amino acid residue(s) of between 0 and 24 consecutive amino acid residues of amino acids 4 to 27 according to Seq.-ID 16 starting with amino acid at position 4 of Seq.-ID 16, said peptide comprises an alpha-amide modification of the C-terminal amino acid residue of said peptide. More preferably the serum albumin peptide is any one according to Seq.-ID 1 to 5 comprising a C-terminal alpha-amide group. Preferably said serum albumin peptide comprises at least one transition metal ion bound by coordination to said peptide, which transition metal ion preferably is selected from the group comprising of copper ions, cobalt ions, nickel ions and iron ions.

Another embodiment of the invention provides a serum albumin peptide containing the C-terminal amino acid of the protein of Seq.-ID 16, which serum albumin peptide is resulting from cleaving off an serum albumin peptide having the amino acid sequence according to Seq.-ID 17 or derivatives or mutants thereof from the protein according to Seq.-ID 16 or naturally occurring derivatives or mutants thereof.

Preferably said C-serum albumin peptide originating form Seq.-ID 16 is resulting from cleaving off a peptide according to any one of Seq.-ID 1 to 5.

In a further aspect the invention provides modifications of the peptides according to the invention, which are mutants showing at least 70 % sequence identity to the corresponding non-mutated sequences.

Further, the present invention relates to peptides having an amino acid sequence as shown in Seq-ID Nos. 19 to 21 and modifications thereof.

In still another aspect the invention provides binding agents specific for a C-terminal alpha-amide present within a protein or peptide. Preferably said binding agent is specific for a peptide according to the invention, and said binding agents recognizes said peptide only, if a C-terminal alpha-amide is present. Preferably said binding agent is an antibody or fragment or derivative thereof, which antibody or fragment or derivative thereof retains its binding specificity for its epitope.

In another aspect the invention provides test kits for performing a method according to the invention. Preferably said test kits comprise a binding agent according to the invention and/or a standard which comprises a peptide according to the invention, and instructions how to use said kit.

In a further aspect the invention provides a method for the manufacture of a peptide according to the invention comprising the step of contacting serum albumin with metal ions of a transition metal and/or, reducing agents, and/or radicals, and/or nitric oxide, resulting in non-enzymatic cleavage of serum albumin and/or loading of serum albumin fragments with nitric oxide and/or transition metal ions. Preferably said method comprising the step of enzymatic or chemical modification of the C-terminal end of said peptide to comprise an alpha-amide is suitable for the manufacture of a peptide according to Seq.-ID 1 to 5, which peptide comprises a C-terminal alpha-amide,

Furthermore the invention provides compositions comprising a peptide and/or a binding agent according the invention for medical uses. Preferably said composition is used for medical purposes as an in vivo nitric oxide delivery vehicle.

Another aspect of the invention provides a method for controlling the presence or absence of a serum albumin peptide according to the invention to treat a condition selected from the list comprising of insulin resistance, glucose intolerance, hyperglycaemia, Type 2 diabetes, ischemia, stroke, hypoxemia, preeclampsia, trauma, coagulation disorders and sepsis, and deprivation of anti oxidants, copper, iron, cobalt or nickel.

In another aspect the invention provides a method for diagnosis of insulin resistance, glucose intolerance, hyperglycaemia, Type 2 diabetes, preeclampsia, trauma, coagulation disorders and sepsis, deprivation of anti oxidants, copper, iron, cobalt, or nickel, or for monitoring of aging, for monitoring of narcosis during surgery, or to detect the use of performance-enhancing substances by an athlete or to detect the application of performance-enhancing substances to animals used in sports, by use of the Ischemia Modified Albumin assay.

In still a further aspect of the invention provides a method for diagnosis of insulin resistance, glucose intolerance, hyperglycaemia, Type 2 diabetes, preeclampsia, deprivation of anti oxidants, deprivation of copper, iron, cobalt or nickel ions, trauma, coagulation disorders and sepsis or for monitoring of aging, for monitoring of narcosis during surgery, or to detect the use of performance-enhancing substances by an athlete or to detect the application of performance-enhancing substances to animals used in sports, by measurement of the binding of copper, cobalt, nickel, or iron ions to serum albumin in a sample from an organism.

Furthermore the invention provides a use of the methods of the invention to replace an Ischemia Modified Albumin assay and/or to replace an assay detecting Haemoglobin A1c (HbA1c).

In still another aspect the invention provides a use of a method according to the invention for diagnostic purposes in combination with an Ischemia Modified Albumin assay and/or an assay detecting Haemoglobin A1c (HbA1c) to improve the specificity and/or selectivity of the diagnosis.

Furthermore, the invention provides new peptides derived from proGIP, i.e. peptides having the sequences shown in Seq-ID. No 19to 21 and their use in the methods and test kits according to the present invention.

### BRIEF DESCRIPTION OF THE DRAWINGS

### Figure 1:

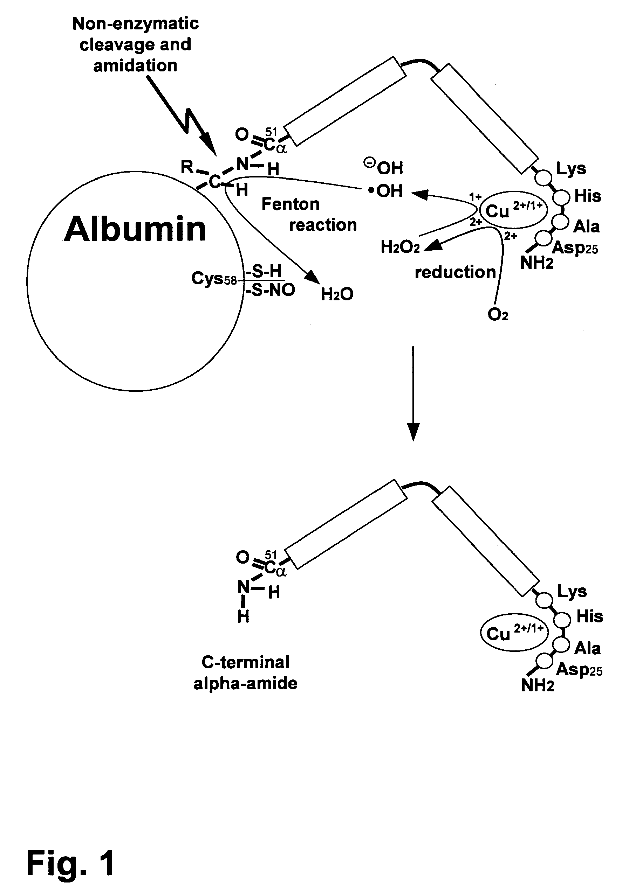

Figure 1 depicts schematically the theory proposed for the origin of the C-terminal alpha-amide of the albumin peptides

### Figure 2:

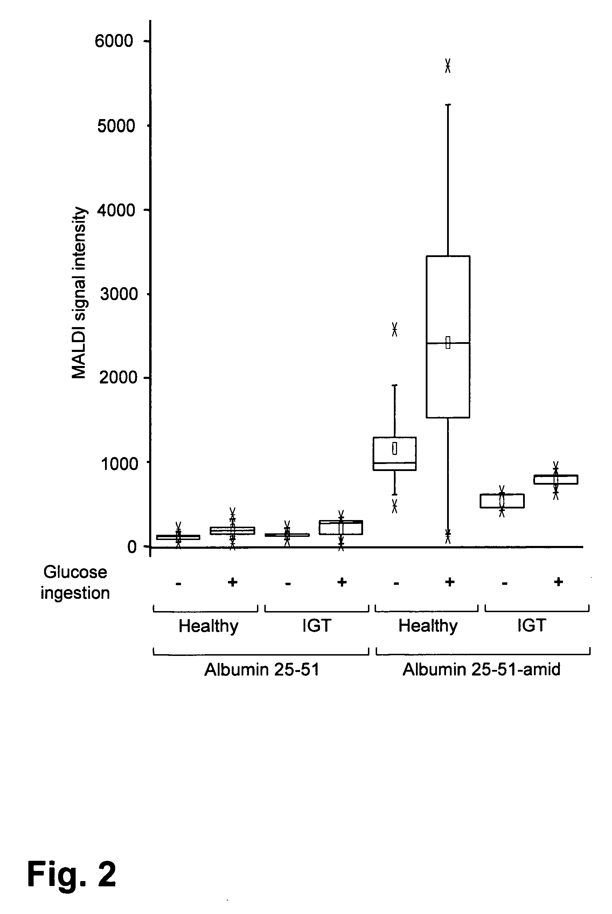

Figure 2 shows box and whisker plots of the serum albumin peptides representing amino acid residues 25 to 51 of human serum albumin with a C-terminal alpha-amide modification (Albumin 25-51-amid, right 4 box-plots) and without such a modification (albumin 25-51; left 4 box-plots). Compared are the mass spectrometric signal intensities (y-axsis) of said peptides as measured in plasma of healthy controls (Healthy) with those signals measured in patients suffering from impaired glucose tolerance (IGT), a pre-form of diabetes. The patients were subjected to an oral glucose tolerance test (Glucose ingestion) and plasma samples obtained before the test ( - ) and samples obtained after 1 and 2 hours ( + ) were measured. The measurement values of the 1 h and the 2 h samples were combined. The box represents the range of 50 % of the measured values, the lower whisker indicates the range of the lowest 25 % of the measured values and the upper whisker indicates the highest 25 % of the measured values. Outliers are indicated by a cross above or below the upper, e.g. the lower whisker.

### Figure 3:

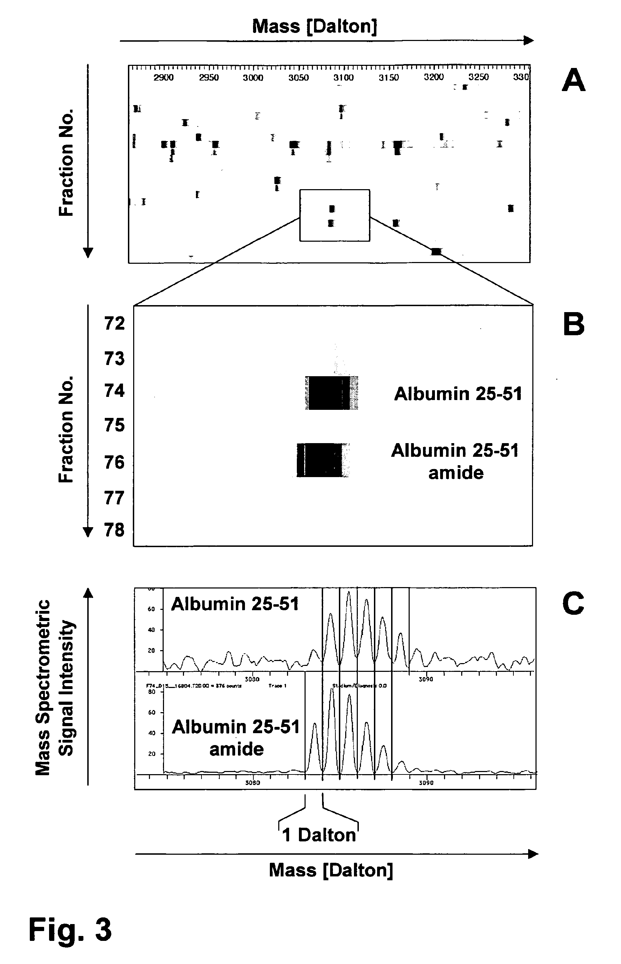

Figure 3 shows results obtained with a plasma sample of a healthy individual. The sample was intentionally chosen to contain two roughly equally signal intensities for the serum albumin fragment representing amino acids 25 to 51 with and without a C-terminal alpha-amide modification. This figure demonstrates that there exist 2 different forms of this serum albumin peptide. Panel A shows a small section of a so called "peptide map", where the x-axis depicts the molecular mass, the y-axis represents the individual fractions analyzed by mass spectrometry and the colour intensity (in grey scale) depicts the mass spectrometric signal intensity. The albumin peptides elute mainly in two different fractions. The non-modified albumin 25-51 elutes predominantly in fraction 74, whereas the C-terminal alpha-amidated albumin 25-51 (albumin 25-51 amide) elutes predominantly in fraction 76. In some samples fraction 75 contains both albumin 25-51 peptides. Panel B shows an enlarged, very small section of the peptide map shown in panel A. It can be seen, that the albumin 25-51 amide has a slightly lower molecular mass of about 1 Dalton as compared to the albumin 25-51 without an amid modification. The signal of albumin 25-51 is spread over a mass range of several Daltons due to the presence of different isotopes, primary carbon isotopes, present in nature, which also result in a several shifts of 1 Dalton each. Panel C finally shows a high resolution mass spectrum of Fraction 74 and 76 of a plasma sample from a healthy individual. Due to the high resolution of this mass spectrum albumin 25-51 peptides with different natural carbon isotope distributions can be resolved. This results in 5 distinct peaks, which are differing in their molecular masses in increments of 1 Dalton. All 5 distinct albumin 25-51 peaks are shifted in the case of the albumin 25-51 amide to molecular mass which is 1 Dalton lower, as compared to the mass spectrum of Albumin 25-51 without an amid modification. The modification of the C-terminus into an alpha-amide results in a reduction of the molecular mass of about 1 Dalton.

### Figure 4:

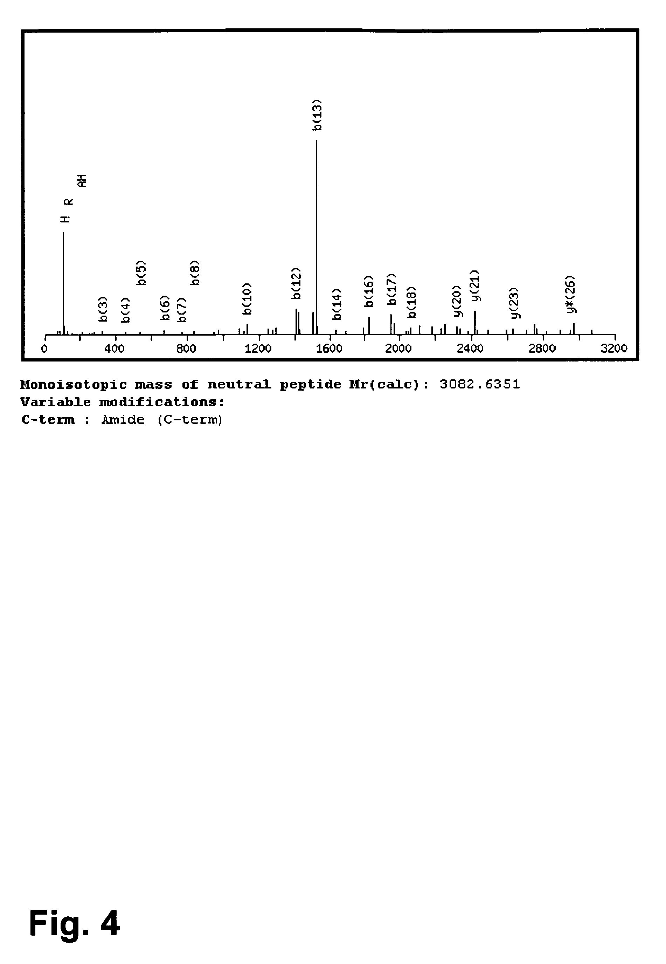

Figure 4 shows the mass spectrometric identification of the albumin 25-51 amide, as described in the examples applying a standard method used in the art to determine peptide sequences including modifications present in these peptides.
. The albumin 25-51 amide is fragmented in a mass spectrometer and the molecular masses of the resulting fragments are determined. Using sequence databases it was calculated, that the determined mass spectrometric-induced fragments of the proposed albumin 25-51 amide indeed originated from albumin 25-51 amide.

### Figure 5:

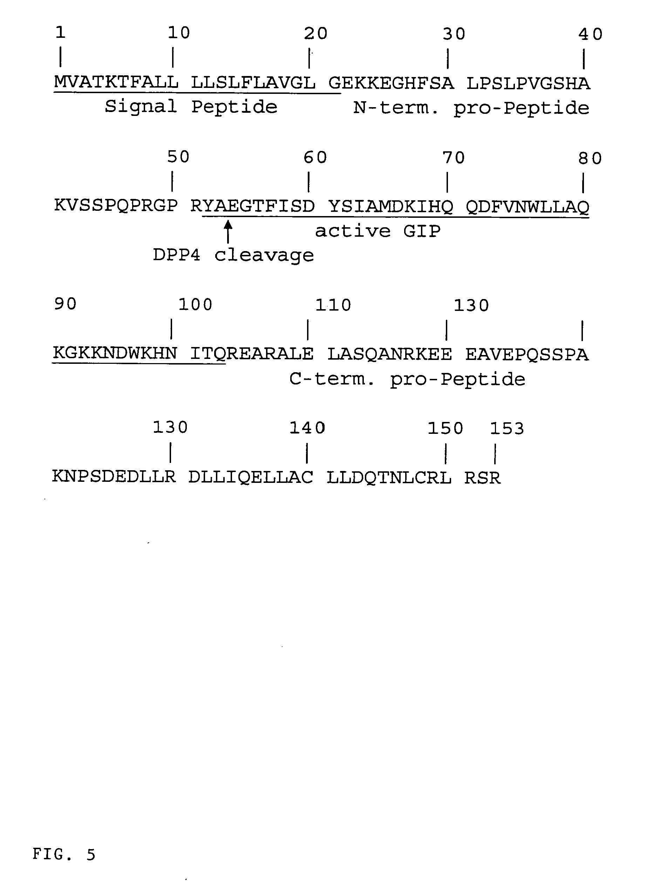

Figure 5 shows the sequence of human proGIP, including its signal sequence (amino acid residue 1 to 21, underlined), the N-terminal pro-peptide of GIP (amino acid residues 22 to 51, not underlined), the active GIP (amino acid residues 52 to 103), underlined; between amino acid residues 53 and 54 is the DPP4 cleavage site, the cleavage of which leads to inactivation of GIP - indicated by an arrow), and the C-terminal pro-peptide of GIP (amino acid residues 104 to 153, not underlined).

### Figure 6:

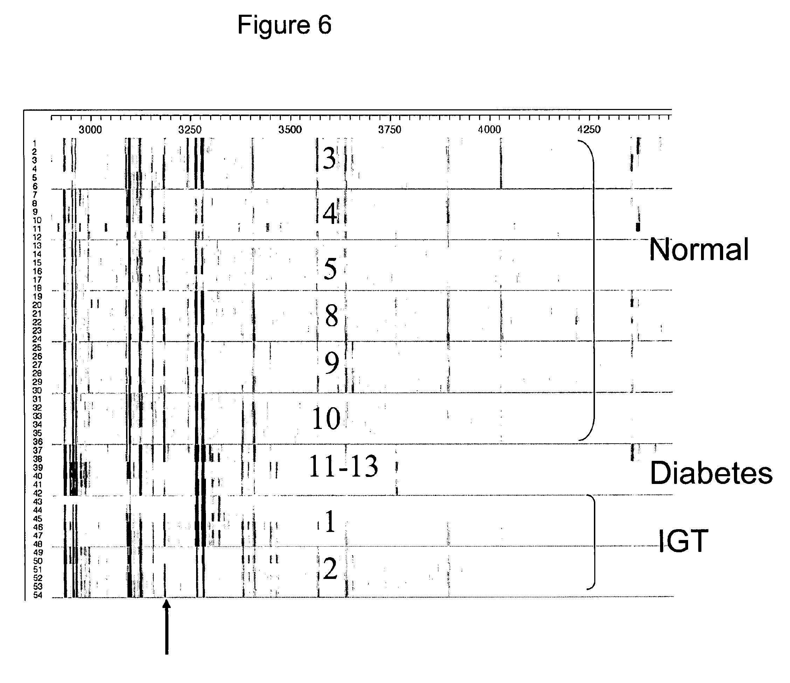

Figure 6 shows the mass spectrometric data for the m/z (mass/charge) range of 3000 to 3250 of the fraction containing the complete N-terminal pro-peptide of GIP (GIP 22-51) having a molecular mass of about 3182 dalton of the plasma samples after 0 h (before ingestion of glucose), after 1 h (=1 h after ingestion of glucose) and after 2 hours. The mass spectrometric signal intensity is depicted by the colour (in grey scale) of the corresponding band. The arrow at the bottom indicates the mass spectrometric signal of the GIP 22-51 peptide.

### DETAILED DESCRIPTION OF THE INVENTION

### Oxidative stress

The term "oxidative stress in an individual" or "oxidative stress in a living organism " according to the invention describes the exposure of structures such as proteins, peptides, carbohydrates, lipids, etc. towards agents, which are capable to oxidize these structures. These oxidising agents, in particular free radicals, usually are produced within the organism and are normally detoxified by antioxidants also present in the organism. If the quantity of the oxidizing agents exceeds the quantity of the antioxidants present in the same location oxidative modification of structures such as proteins, peptides, carbohydrates and lipids can take place. Oxidating agents usually are instable substances with an unpaired free electron, which is very reactive with other structures, whereas antioxidants usually can provide such an unpaired free electron without becoming instable. Manny antioxidants such as vitamins C and E, carotenoids, selenium, are present in food products; others are synthesized by the organism itself or are intentionally applicated to the organism, for example medicaments. Oxidative stress is imposed on cells as a result of 1) an increase in oxidant generation, 2) a decrease in antioxidant protection, or 3) a failure to repair oxidative damage or any combination of items 1) to 3). Examples of oxidative stress among others are amino acid oxidation products such as methionin sulfoxide, ortho-tyrosine (o-tyr) and dityrosine, chlorotyrosine and nitrotyrosine, chemical modifications of protein following carbohydrate or lipid oxidation, such as N epsilon- (carboxymethyl) lysine and N epsilon- (carboxyethyl) lysine, and malondialdehyde and 4-hydroxynonenal adducts to amino acids, the amino acid cross-link pentosidine, the imidazolone adducts formed by reaction of 3-deoxyglucosone or methylglyoxal with arginine, and the imidazolium cross-links formed by the reaction of glyoxal and methylglyoxal with lysine residues in protein (Ann N Y Acad Sci, 1998, 854:277-90). Furthermore lipids can be modified as a consequence of oxidative stress, for example by lipid peroxidation and DNA damage due to oxidative stress results in 8-Hydroxy-2'-deoxyguanosine (8-OhdG) (Biomarkers of Nutritional Exposure and Nutritional Status, 2003, Supplement, page 933S-940S).

### Methods to determine oxidative stress

There are known various methods in the art for detecting oxidative stress. For example the thiobarbituric acid-reactive substance (TBARS) assay, a spectrophotometric assay measuring a chromogen formed from the reaction of thiobarbituric acid (TBA) with malondialdehyde (MDA), which is a product of lipid peroxidation. Further assays to determine oxidative stress are the measurement of breath hydrocarbons such as pentane and ethane formed by peroxidation of (n-6) fatty acids and (n-3) fatty acids, measuring of LDL resistance to exogenously in vitro added oxidants, measurement of F2 isoprostanes which originate from peroxidation of arachidonic acid, the ferrous xylenol orange (FOX) assay or assays measuring plasma lipid hydroperoxides by chemiluminescence, oxygen radical absorbance capacity (ORAC) assays, etc. Biomarker for DNA oxidation are 8-hoydroxy-2'-deoxyguanosine (8-OhdG) and auto-antibodies to oxidized DNA. The comet assay measures breaks in supercoiled DNA as an indicator of oxidative DNA-damage (Biomarkers of Nutritional Exposure and Nutritional Status, 2003, Supplement, page 933S-940S). Biomarkers for protein oxidation are protein carbonyls, which can be detected using dinitrophenylhydrazine in a colorimetric procedure (Meth Enzymol, 1990, 186:464-78). Protein carbonyls can also be determined by enzyme-linked immuno sorbent assays (ELISA) and Western blotting (Meth Enzymol, 1999, 300:106-11; Meth Enzymol, 1994, 233:346-63). Furthermore "Analysis of advanced Oxidation Protein Products", AOPP, can be done to spectrophotometrically measure oxidative stress on proteins (Kidney Int, 1996, 49:1304-13).

As used herein, the term "individual" or "subject" or "living organism" which is used herein interchangeably refers to an individual or a subject or a living organism in need of a therapy or prophylaxis or suspected to be afflicted with a condition or disease mentioned herein. Preferably, the subject or individual or living organism is a vertebrate, even more preferred a mammal, particularly preferred a human.

The method according to the present invention is preferably conducted by collecting a sample from the individual and measuring in vitro the amount of the least one protein according to the present invention using appropriate means.

The term "precursor peptide or protein" as used herein refers to peptides or proteins occurring in nature, i.e. naturally occurring peptides or proteins encompassing full-length proteins and proteins or peptides which may be derived from pre and/or pro-forms of said proteins and the peptides cleaved off the mature proteins. Further, said term comprises naturally occurring fragments of said peptides or proteins. Naturally occurring means that said peptides and proteins and fragments thereof are detectable in individuals or subjects without additional artificial impact thereon.

### Peptides and proteins

The term "peptide" refers to substances consisting of two or more, preferably 3 or more, preferably 4 or more, preferably 6 or more, preferably 8 or more, preferably 10 or more, preferably 13 or more, preferably 16 more, preferably 21 or more amino acids joined covalently by peptide bonds. The term "protein" refers to large peptides, preferably to peptides with at least 160 amino acids, but in general the terms "peptides" and "proteins" are synonyms and are used in this application as synonyms. The terms "peptide" and "protein" according to the invention include substances containing not only amino acids, but also substances also containing non-amino acid constituents and include substances containing only peptide bonds as well as substances also containing other bonds, e.g. ester, thioether or disulfide bonds.

Further, the term "peptide" and "protein" encompasses modifications thereof like derivatives and polymorphic forms and fragments thereof.

Peptides and variants thereof having less than about 100 amino acids, and generally less than about 50 amino acids, may be generated by synthetic means, using techniques well known to those of ordinary skill in the art. For example, such peptides may be synthesized using any of the commercially available solid-phase techniques, such as the Merrifield solid-phase synthesis method, where amino acids are sequentially added to a growing amino acid chain. See Merrifield, 1963, J. Am. Chem. Soc. 85, 2149-2146. Equipment for automated synthesis of polypeptides is commercially available from suppliers such as Perkin Elmer/Applied BioSystems Division (Foster City, CA, USA), and may be operated according to the manufacturer's instructions. It is also possible to chemically synthesize C-terminal alpha-amidated peptides.

Within certain specific embodiments, a peptide or protein may be a fusion peptide or fusion protein that comprises a fusion partner, which fusion partner may, for example, assist in expressing the peptide or protein (an expression enhancer) at higher yields than the non-fused recombinant peptide. Other fusion partners may be selected so as to increase the solubility of the peptide or protein or to enable the peptide or protein to be targeted to desired intracellular compartments. Still further fusion partners include affinity tags, which facilitate purification and/or detection of the peptide or protein.

Fusion peptides or fusion proteins may generally be prepared using standard techniques, including chemical conjugation. Preferably, a fusion peptide or fusion protein is expressed as a recombinant protein, allowing the production of increased levels, relative to a non-fused peptide or protein, in an expression system. Briefly, DNA sequences encoding the peptide or protein components may be assembled separately, and ligated into an appropriate expression vector. The 3' end of the DNA sequence encoding one peptide or protein component is ligated, with or without a DNA sequence encoding a peptide linker, to the 5' end of a DNA sequence encoding the second peptide or protein component so that the reading frames of the sequences are in phase. This permits translation into a single fusion peptide or fusion protein that retains the biological activity of both component peptides or proteins. A peptide linker sequence may be employed to separate the first and second peptide or protein components by a distance sufficient to ensure that each peptide or protein folds into its secondary and tertiary structures. Such a peptide linker sequence is incorporated into the fusion peptide or fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second peptides or proteins; and (3) the lack of hydrophobic or charged residues that might react with the peptide or protein functional epitopes. The linker sequence may generally be from 1 to about 50 amino acid residues in length, preferably 1 to 40, 1 to 30, 1 to 20, 1 to 10 or 1 to 5 amino acid residues in length. Linker sequences are not required when the first and second peptides or proteins have non-essential N-terminal amino acid regions that can be used to separate the functional domains and prevent steric interference. If the fusion peptide is prepared by chemical conjugation or during chemical synthesis it is possible, that the linker is completely or partially comprising non-amino acid residue structures functioning as linker. These structures preferably are made from carbon-hydrogen and/or carbon-nitrogen and/or carbon-oxygen and/or carbon-sulfur structures.

### Modifications of peptides and proteins

Modifications of peptides and proteins described herein may comprise modifications due to posttranslational modifications, chemical modifications, enzymatic modifications and modifications due to other mechanisms. Examples of possible modifications include but are not limited to: glycosylation, phosphorylation, sulphatation, pyroglutamate modification, cystein-disulfide bridges, methylation, acetylation, acylation, farnesylation, formylation, geranylgeranylation, biotinylation, stearoylation, palmitylation, lipolyation, C-mannosylation, miristoyliation, amidation other than at the C-terminal amino acid residue, deamidation except for the C-terminal alpha-amide, methylation, demethylation, carboxylation, hydroxylation, iodination, oxidation, pegylation, prenylation, ADP-ribosylation, addition of lipids, of phosphatidylinositol, of glycosylphosphatidylinositol (GPI)-anchor, of pyridoxal phosphate, modification of cysteine residues resulting in carboxyamidomethylcysteine, resulting in carboxymethylcysteine, or resulting in pyridylethylcysteine, modification of lysine residues resulting in liponic acid, modification of glutamic acid resulting in pyroglutamic acid, etc.

Modifications of peptides or proteins described herein may comprise unusual amino acids, chemically or enzymatic modified amino acids etc. including, but not limited to: alpha-amides, C-terminal alpha-amides, alpha amino butyric acid, beta amino butyric acid, beta amino iso-butyric acid, beta alanine, gamma butyric acid, alpha amino adipic acid, 4-amino benzoic acid, amino ethyl cysteine, alpha amino penicillanic acid, allysine, 4-carboxy glutamic acid, cystathionine, carboxy glutamic acid, carboxy amido methyl cysteine, carboxy methyl cysteine, cysteine acid, citrulline, dehydroalanine, di-amino butyric acid, dehydro amino-2-butyric acid, ethionine, glycine-proline di-peptide, 4-hydroxyproline, hydroxylysine, hydroxyproline, homoserine, homo cysteine, histamine, iso-valeine, lysinoalanine, lanthionine, norvaline, norleucine, ornithine, 2-pipiridine-carboxylic acid, pyroglutamic acid, pyrrolysine, proline-hydroxy proline di-peptide, sarcosine, 4-selenocysteine, syndesine, thioproline, etc. Further examples can be found in databases such as the "Delta Mass" database searchable at the website of the ABRF, the "Association of Biomolecular Resource Facilities": http://www.abrf.org/index.cfm/dm.home?AvgMass=all.

In addition, the modifications of the proteins and/or peptides may be in form of derivatives or polymorphic form forms or fragments of the peptides or proteins. DerivativesThese kind of peptides or proteins are primary characterized by modifications of one or more amino acid residues or modifications of the peptide bond structure, whereas and polymorphic forms of peptides or proteins are characterized by differences in the amino acid sequence, which differences may comprise changes of one or more than one amino acid residues, deletion of one or more than one amino acid residues, and insertions of one or more than one amino acid residues (mutants of the peptide or protein). It is possible, that a modified peptide or protein at the same time is a derivative and/or a polymorphic formpeptide comprising posttranslational modifications and/or fragmentunusual amino acids and/or chemically or enzymatic modified amino acids and/or a polymorphic forms and/or fragments of another peptide or protein.

With derivatives of peptides and/or proteins are meant all kind of peptides and/or proteins and/or fragments thereof, including peptides and/or proteins comprising posttranslational modifications, chemical modifications, enzymatic modifications and modifications due to other mechanisms. Derivatives of peptides may comprise amino acid residues different from the standard set of 20 amino acids and/or may comprise peptidomimetic structures.

With polymorphic forms of peptides and/or proteins are meant variations of the sequences due to mutations present in nature or due to mutations caused by experimental or random manipulation of the sequence using techniques such as molecular biology, genetics or chemistry (nucleic acid or peptide synthesis).

Furthermore polymorphic forms of peptides and/or proteins preferably have 70 % sequence identity, preferably 75 %, preferably 80 %, preferably 85 %, preferably 90 %, preferably 95 %, preferably 97 % and preferably have 99 % sequence identity with each other, as determined by sequence alignment. An amino acid residue or nucleotide is regarded as identical in two sequences, if within a sequence alignment of these two sequences this amino acid residue or nucleotide is placed at the same position. If for example a first hypothetical sequence "ACDEF" and a second hypothetical sequence "ACEF" with a deleted "D" are aligned, there would be inserted a gap into the second sequence between "F" and "H" resulting in "AC-EF" thereby enabling a better alignment for of the second sequence to the first sequence. Consequently, in the resulting alignment there are now 4 out of 5 positions occupied by identical amino acid residues resulting in a sequence identity of the first to the second sequence of 80 %. Methods how to calculate sequence alignments are given below.

### Preparing of sequence alignments and calculation of sequence homology and sequence identity

To calculate the homology of sequences computer programs such as the GCG software suite (Genetics Computer Group, University of Wisconsin, Madison, WI, USA), including GAP (Nucleic Acids Res. 12:387-95), BLASTP, BLASTN, FASTA (J Mol Biol. 215:403-10) or the well-known Smith Watermann-algorithm can be used. Preferred parameter used for amino acid sequence comparisons or alignments comprise the algorithm of Needleman and Wunsch (J Mol Biol. 48:443-53), the BLOSUM 62 matrix (Proc Natl Acad Sci U S A. 89:10915-9), a gap penalty of 12, a gap length penalty of 4 and a threshold of similarity of 0. The GAP software is also suitable to be used with the parameters mentioned. The stated parameters are the default parameter for amino acid comparisons, wherein gaps at the end of a sequence do not decrease the homology value. If very short sequences such as 8 to 20 amino acid residues long sequences are compared it may be necessary to increase the expectation value up to 100 000 and to reduce the word length down to 2. Further suitable algorithms are the use of gap opening penalties, gap extension penalties and the use of matrixes found in the program handbook, Wisconsin Package, version 9, from September 1997. The choice of the most suitable algorithms depends on the kind of comparisons to perform. If two sequences are compared the GAP or the Best Fit algorithms are preferred, if one sequence is compared to a sequence database the FASTA and BLAST algorithms are preferred. A sequence identity of 70 % is also termed a homology of 70 %. Identity means, that at the same position within both sequences of the alignment the same amino acid residue or nucleotide is present.

### Peptidomimetics of peptides and proteins

Peptides and proteins described herein can be prepared or synthesized to represent partially or completely peptidomimetics. This allows designing peptides with more defined properties. For example D-amino acids could be used to prevent proteolysis. "Peptidomimetics" according to the invention are structures, which mimic the function of amino acids or amino acid sequences. "Peptidomimetics" or "mimetics of peptides" often have additional properties such as increased resistance to proteolysis. Peptidomimetics can replace some or all peptide bonds by other kinds of covalent bonds, which can connect amino acids. Peptidomimetics among others can comprise modifications of amino acids such as alpha-C alkylation, alpha-N alkylation, etc, dipeptide analogues (two amino acid side chains which are connected by covalent bonds) or modifications of the peptide backbone, especially change from L- to D-amino acids, inverse N- to C-sequence, amide bond isosteres, etc. Further examples of peptidomimetics are spiegelmers® (NOXXON Parma AG, Berlin, Germany) or beta amino acids.

### Nucleic acids, complementary and degenerated nucleic acids and derivatives

With nucleic acids or nucleic acid sequences are meant all kinds of deoxyribonucleic acids (DNA) and ribonucleic acids (RNA) or combinations thereof regardless if these nucleic acid molecules are single or double-stranded, linear, circular or branched. It will be understood that when a nucleotide sequence is represented by a DNA sequence ( i.e., A, T, G, C), this also includes an RNA sequence (i.e., A, U, G, C) in which "T" replaces "U". Preferably the nucleic acid molecules are purified nucleic acid molecules which means that the preparation contains at least 50 %, preferably at least 60 %, preferably at least 70 %, preferably at least 80 %, preferably at least 90 %, preferably at least 95 %, preferably at least 99 %, preferably more than 99 % of a certain nucleic acid molecule or of a certain mixture of more than one certain nucleic acid molecule. Examples of nucleic acid molecules are genomic DNA, cDNA (complementary DNA), mRNA (messenger RNA), recombinantly produced nucleic acid molecules, chemically synthesized nucleic acid molecules or nucleic acid molecules generated by enzymatic methods for example by use of polymerase chain reaction (PCR) or nucleic acid molecules purified from natural sources such as prokaryotic or eukaryotic cells, viruses, tissues, organs or whole organisms such as bacteria, yeast, insects, worms, etc. using methods known in the art. Furthermore nucleic acid molecules according to the invention can be partially or completely represent derivatives of nucleic acids which derivatives comprise nucleotides or nucleotide-like molecules not found in nature such as phosphorothioates, peptide nucleic acids (PNAs), N3', P5'-phosphoramidates, morpholino phosphoroamidates, 2'-O-methoxyethyl nucleic acids, 2'-fluoro-nucleic acids, arabino-nucleic acids, locked nucleic acids (LNA, ribonucleotides containing a methylene bridge that connects the 2'-oxygen of ribose with the 4'-carbon).

### Vectors

Nucleic acid sequences can be part of a vector. A vector can be circular or linear or branched, single or double stranded, can be composed of ribonucleic acids, deoxyribonucleic acids or combinations thereof. The vector can be a naked nucleic acid, a nucleic acid associated with for example proteins, lipids, liposomes, calcium-phosphate precipitates, or other substances or combinations thereof. The vector can also be a virus or cell comprising said nucleic acids. The vector can be suitable to enhance or decrease the amount of for example N-terminal or C-terminal proGIP, or serum albumin fragments and/or modifications thereof. Vectors increasing the amount of said peptides and/or proteins or modifications thereof are for example transient or stable expression vectors. Vectors decreasing the amount of said peptides and/or proteins or modifications thereof for example comprise nucleic acid sequences coding for example for serum albumin sequence-specific antisense-nucleic acids, ribozymes, triplex-forming nuclei acids, RNAi-molecules, etc. The vector can be present in the form of a virus such as a retrovirus, an adenovirus, a Bacculovirus, a phage or other viruses known in the art. The virus can be a viable virus or a virus needing a helper-virus for proliferation and/or infection. The vector can be unspecific or specific for certain species such as humans, mice, rats, etc. or groups of species such as mammals, rodents, etc., or certain organs or tissues such as muscle-tissue, pancreas, liver, fat-tissue, etc. certain types of cells such as fat cells, islet cells, muscle cells, cells of the pancreas, neuronal cells, cells from the gastrointestinal tract, etc. or specific for bacteria, yeasts, insect cells, cell lines such as COS-cells or CHO-cells. The specificity of the vector can be due to specific promoter-nucleic acid sequences which are only active in specific cell types or species, or the vector can be specific by mechanisms targeting the vector to specific cells, or a promoter present in the vector can be activated or can be inactivated by substances given to the individual or cell comprising said vector, etc.

### Host cells

"Host cell" does not mean a cell from the host of a xenograft, but host cells means cells comprising a nucleic acid and/or a vector as described in the previous chapters. The nucleic acid or vector can be present in transient or in stable form in the cell and the nucleic acid or vector can be present in the cytosol or certain organelles of the cell such as the nucleus, the mitochondria or the cytosol. The vector sequence or fragments thereof can be present in the cytosol for example in the form of a plasmid or the vector sequence can be partially or completely integrated into the genome of the cell or the genome of the mitochondria, or into a plasmid present in the cell by site (sequence) specific, by random or by other mechanisms.

### Binding agents, antibodies and derivatives

Another embodiment of the invention refers to on the one hand to binding agents specific for proteins or peptides having a C-terminal alpha amide modification and which can distinguish between said peptide or protein having a C-terminal alpha-amide and the same peptide not having said modification. Particularly, the binding agents are specific for serum albumin peptides, especially specific for serum albumin peptides comprising a C-terminal alpha-amide modification. In addition the invention comprises binding agents specific for a fragment, or modifications of the mentioned peptides. In particular, the binding agents according to the present invention specifically bind to at least one neo-epitope of a peptide according to any one of Seq.-ID 1 to 5 with the proviso that they do not bind to a peptide according to Seq.-ID 6 to 11. Preferably these binding agents are specific for human serum albumin peptides and for serum albumin peptides originating form animal species used in sports such as horses and dogs. Binding agents according to the invention are among others receptors specific for these peptides, phages binding specific to these peptides, spiegelmers® binding specific to these peptides, small organic molecules binding specifically to these peptides, particles or surfaces coated with substances specifically binding to these peptides, such as receptors, phages, small organic molecules, spiegelmers®. Preferably specific binding agents comprise specific antibodies binding said peptides and/or proteins or fragments or modifications thereof. Also included are fragments of said antibodies as long as these fragments specifically bind to said peptides and/or to said proteins or to said fragments or to said modifications. Preferably the binding agents specific for said peptides and/or proteins or fragments or derivatives thereof are antibodies such as an IgG-, IgA-, IgE-, IgD- or an IgY-antibodies originating from any species in purified form, or present for example in the form of a cell culture supernatant, an antiserum, as ascites fluid or contained within eggs. The antibodies preferably can originate from humans, mice, rats, goats, donkeys, cows, horses, or other mammals or from eggs preferably from chicken eggs, or the antibodies can be produced using recombinant techniques know in the art. The antibody can be a monoclonal, an oligoclonal or a polyclonal antibody. The antibody preferably can be a purified specific antibody, a purified specific antibody further comprising non-specific other antibodies or other non-antibody proteins, an antibody prepared from whole anti-serum, prepared from cell culture supernatant, prepared from eggs or prepared from ascites fluid or an unpurified antibody, preferably unpurified anti-serum, unpurified cell culture supernatant, unpurified ascites fluid or an unpurified recombinant antibody. Preferably a purified antibody or antibody fragment or derivative according to the invention comprises at least 30 %, preferably at least 40 %, at least 50 %, 60 %, 70 %, 75 %, 80 %, 85 %, 90 %, 95 %, or preferably at least comprises 98 % of said antibodies and/or antibody fragments and/or derivatives thereof. Unpurified cell culture supernatant or anti serum preferably contains at least 0.5 %, at least 1 %, at least 2 %, at least 5 %, at least 10 %, at least 15 % of said antibodies and/or antibody fragments and/or derivatives thereof.

With specific binding agents are meant for example antibodies, which bind specifically to a protein or peptide ligand such as a serum albumin peptide according to the invention, especially a serum albumin peptide comprising a C-terminal alpha-amidation, or with specific binding agents are meant nucleic acids, which bind specifically to a nucleic acid sequence coding for a peptide according to the invention or nucleic acid sequence complementary or homologous to said nucleic acid sequence. The specificity of the binding of a binding agent, such as an antibody or a nucleic acid for example, can be tested by standard western blotting, northern blotting, reverse transcriptase-polymerase chain reaction (RT-PCR) or other methods known in the art. As samples there can be used for example cells transfected with a nucleic acid coding for a serum albumin fragment for which the antibody to be tested is supposedly specific. As negative control the same but not transfected cell line or a cell line transfected with a non-serum albumin fragment can be used. For determining the specificity of antibodies cell lysates from both cell lines are tested in a western blot. A specific binding agent, such as an antibody, recognizes a band of the correct molecular weight only in the lysate from the cells transfected with the serum-albumin fragment but not in the lysate from a negative control, e.g. the non-transfected cells or the cells transfected with a non-serum albumin fragment. For determining the specificity of nucleic acids, RNA can be purified from both cell lines and tested in for example northern blots. A specific binding nucleic acid recognizes a band of the correct molecular weight only in the RNA sample of the transfected cell line but not in the RNA from the negative control, resulting in a signal of the appropriate molecular weight on the northern blot. However, as known in the art, there can also be signals on the northern blot in the negative control, but the signal in the negative control is clearly lower, if the binding agent is specific for the ligand transfected into the cell line. The specificity can be further tested by increasing the stringency of the hybridization and/or washing steps of the northern blots, which should gradually reduce the signal in the negative control, whereas the signal of the serum albumin-transfected cells remains present.

The invention furthermore comprises derivatives of antibodies such as an antibody comprising parts of antibodies originating from different species, for example humanized antibodies, which for example comprise the antigen binding domain of a murine antibody and other parts of the antibody or antibody fragment originate from human antibodies. The invention also includes fragments and derivatives of antibodies as long as these antibodies still specifically bind to the serum albumin peptides of the invention and/or to fragments and/or derivatives and/or polymorphic forms thereof. Examples of suitable antibody fragments are F(ab)2, Fab, F(ab')2, Fab', Fv and single chain Fv (scFv) antibodies. Further derivatives of antibodies according to the invention are antibodies or fragments of antibodies covalently or non-covalently bound to other substances such as organic compounds, inorganic compounds, peptides or proteins. Examples for such derivatives of antibodies are antibodies labeled with radionuclides, toxins, enzymes, fluorophores, chromophores, biotin or other organic compounds, fluorescent proteins, dyes, or label sequences such as the label sequences described in previous chapters.

### Compound and/or composition

The term "compound and/or composition" according to the invention relates to all kinds of substances including small organic or inorganic molecules, chemically, enzymatic or recombinantly synthesized molecules, such as nucleic acids, peptides, proteins, carbohydrates or lipids, synthetic molecules or molecules found in nature or molecules isolated from materials or organisms found in nature, as well as molecules found in nature with altered structures or with sequences comprising deletions, insertions and mutations of the sequence and combinations or mixtures thereof. Compositions according to the invention comprise at least one compound and additional substances such as saline, diluents, solvents, substances ensuring an appropriate pH, osmolarity or viscosity, additives for preservation, staining, flavouring or scenting, filling substances, anti-microbial, anti-fungal or other preserving agents, substances protecting the compound or composition form heat, freezing, day light, UV-light or other radiation, or other environmental factors, substances preventing aggregation of the compounds or compositions, or protecting the compounds or compositions from sticking to the container in which they are stored or placed during application, formulations necessary for specific routes of applications such as gastric acid resistant capsules, sterile solutions for injections or infusions, or plasters for transdermale application of compounds or compositions etc.

### Compounds and compositions

Another embodiment of the invention are compositions comprising at least one compound, which compositions preferably are suitable for medical purposes, preferably suitable as pharmaceutical compositions, preferably suitable as medicaments for therapy and/or prophylaxis or compositions suitable as diagnostics. These compositions comprise compounds such as the peptides listed in the sequence protocol or fragments or modifications of said peptides, nucleic acids coding for said peptides, or fragments or derivatives of said nucleic acids, or said nucleic acids complementary to said nucleic acids, vectors comprising said nucleic acids, host cells comprising said nucleic acids or said vectors, binding agents or antibodies specific for said peptides, especially specific for C-terminal alpha-amidated peptides, or fragments or derivatives of said binding agents or antibodies.

The pharmaceutical compositions of the invention can be used for therapy, prophylaxis, diagnosis and prognosis, of a condition or disease selected form insulin resistance, glucose intolerance, hyperglycemia, Type 2 diabetes, preeclampsia, trauma, coagulation disorders, sepsis, and deprivation of anti oxidants, copper, iron, cobalt or nickel, or for monitoring of aging, for monitoring of narcosis during surgery, or to detect the use of performance-enhancing substances by athletes or to detect the application of performance-enhancing substances to animals used in sports

The pharmaceutical compositions can be administrated orally, sublingually, injected intra venous, injected subcutaneously, injected into muscle, fat, pancreas, or other tissues, administered topical onto the skin or onto a mucosa, inhaled as aerosol into the lungs or administrated rectal or through other routes. The pharmaceutical composition can be supplied as tablets, pills, capsules, powders, granulates, salves, plasters releasing substances transdermal to the individual, tinctures, uvula, suspensions, solutions, which solutions preferably are dissolved in sterile physiological sodium chloride solutions for use in injections or as infusions, etc. The pharmaceutical compositions can comprise additives such as filling additives, antimicrobial additives, anti-fungal additives, or other preservative additives, coloring additives, flavoring additives, smelling additives, protective or stabilizing additives, etc. Preferred protective or stabilizing additives for example are substances, which inhibit degradation of peptides or proteins such as for example protease inhibitors or carrier proteins to protect the peptides and proteins from degradation, aggregation or from loss of activity. The composition can be applied to an individual in need once or repeatedly. It can be applied one or more times a day, weekly, monthly or in longer time intervals. The composition can be used alone or in combination with other compositions, compounds, substances or drugs. Combined compositions can have less than additive, can have additive and can have synergistic effects on the individual treated or tested. The composition can comprise up to 3 µg, up to 10 µg, up to 30 µg, up to 100 µg, up to 300 µg, up to 1 mg, up to 30 mg, up to 300 mg, up to 1 gram of an serum albumin peptide with a C-terminal amid group or a nitric oxide binding side, and/or nucleic acid coding for said peptide, and/or a binding agent specifically binding to said peptide, and/or an antibody specifically binding to said peptide, or of a fragment, derivate or polymorphic form of the said, or combinations thereof.

If the compound or composition is used as a diagnostic it can be used alone or in combination with other diagnostics or diagnostic methods. The result of the diagnosis by combination of two or more compositions and/or diagnostic methods can improve the reliability of the diagnosis less than additively or it can improve it additively or it can improve the diagnosis synergistically. The composition can be used for diagnosis to prove the presence or to prove the absence of a disorder, preferably chosen from the list comprising insulin resistance, glucose intolerance, hyperglycaemia, Type 2 diabetes, preeclampsia, trauma, coagulation disorders and sepsis, deprivation of anti oxidants, copper, iron, cobalt, or nickel, or for monitoring of aging, for monitoring of narcosis during surgery, or to detect the use of performance-enhancing substances by an athlete or to detect the application of performance-enhancing substances to animals used in sports.

If the composition is used as a diagnostic it may be used in combination with the IMA-test or with a diagnostic test measuring glycosylated hemoglobin (HbA1c) to improve the specificity or selectivity of the diagnosis according to the invention. If the composition is used as a diagnostic it may be used to replace the diagnosis by an IMA-test or it might replace the diagnosis by and test for glycosylated hemoglobin, or it might serve to improve the specificity or selectivity of the IMA-test or it might serve to improve the specificity of a test for glycosylated hemoglobin.

### Suitable methods to analyze the sample

Generally all methods suitable to detect and analyze the peptides and/or proteins present in a sample can be used in the methods of the invention and can be used to determine the peptides and/or proteins and/or nucleic acids, preferably serum albumin peptides and/or nucleic acids coding for the said or the corresponding complementary nucleic acids. Preferably mass spectrometric methods, protein chip assays, immunology and molecular biology methods can be used.

### Qalitative and quantitative measurements

With determining of peptides, proteins, fragments or modifications thereof is meant the qualitative or quantitative measurement of these substances. Depending on the sensitivity of the method used, the determination may have as a result that a substance is not present in a sample (e.g. it is present below the detection limit of the method used). If the quantity is determined the result may be a relative or an absolute value, depending on the method used.

Suitable mass spectrometric methods among others are matrix-assisted laser desorption ionisation (MALDI), continuous or pulsed electrospray ionization (ESI) and related methods such as ionspray or thermospray or massive cluster impact (MCI). The ion sources can be matched with detection formats including linear or non-linear reflection time-of-flight (TOF), single or multiple quadrupole, single or multiple magnetic sector, fourier transform ion cyclotron resonance (FTICR), ion trap, and combinations thereof, e.g. ion-trap/time-of-flight. For ionization, numerous matrix/wavelength combinations (MALDI) or solvent combinations (ESI) can be used. Other mass spectrometric methods suitable are for example fast atom bombardment (FAB) mass spectrometry, Surface Enhanced Laser Desorption/lonization (SELDI) mass spectrometry, isotope coded affinity tag (ICAT) mass spectrometry, iTRAQ mass spectrometry (Applied BioSystems, Foster City, CA, USA) or affinity mass spectrometric methods.

Furthermore suitable immunologic methods among others are enzyme linked immuno assays (ELISA), sandwich, direct, indirect, or competitive ELISA assays, enzyme-linked immunospot assays (ELISPOT), radio immuno assays (RIA), flow cytometry assays (FACS = fluorescence activated cell sorting), immunohistochemistry, Western blot, fluorescence resonance energy transfer (FRET) assays, protein-chip assays using for example antibodies, antibody fragments, receptors, ligands, or other agents binding the peptides of the invention, preferably to the serum albumin peptides of the invention, preferably to the serum albumin peptides of the invention with a C-terminal alpha-amidation, preferably to the serum albumin peptides according to Seq.-ID 1 to 5.

Among others Northern or Southern blot hybridization, nucleic acid dot- or slot-blot hybridization, in situ hybridization, nucleic acid chip assays (for example using RNA, DNA or other nucleic acids to specifically capture the nucleic acids of interest), polymerase chain reaction (PCR), reverse transcriptase PCR (RT-PCR), real time PCR (taq-man PCR) among others can be used as molecular biology methods to detect the nucleic acids of the invention.

Further methods such as nuclear magnetic resonance (NMR), fluorometry, colorimetry, radiometry, luminometry, or other spectrometric methods, liquid chromatography, capillary chromatography, thin-layer chromatography, plasmon-resonance (e.g. BIACORE), one- or two-dimensional gel electrophoresis, etc. can be used to detect the peptides and/or proteins and/or nucleic acids, preferably those corresponding the serum albumin peptides of the invention, preferably to serum albumin peptides with a C-terminal alpha-amidation, preferably to the serum albumin peptides according to Seq.-ID 1 to 5.

### Test kits

A further aspect of the invention are test kits for determining the absolute concentration or the relative concentration or the presence or absence of the serum albumin peptides of the invention, preferably to serum albumin peptides with a C-terminal alpha-amidation, preferably to the serum albumin peptides according to Seq.-ID 1 to 5 and corresponding nucleic acid or a fragments or derivatives or polymorphic forms thereof. The test kit can be used to diagnose the absence or presence of disorders selected from the list comprising insulin resistance, glucose intolerance, hyperglycaemia, Type 2 diabetes, preeclampsia, trauma, coagulation disorders and sepsis, deprivation of anti oxidants, copper, iron, cobalt, or nickel. The test kit further can be used to predict the occurrence or to predict the grade of said disorder and/or to predict and/or monitor the success of a therapy for said disorder and/or to predict and/or to monitor aging, or to monitor narcosis during surgery, or to detect the use of performance-enhancing substances by an athlete or to detect the application of performance-enhancing substances to animals used in sports. Preferably the test kit can be used to early diagnose and/or predict said disorder, preferably before the diagnosis is possible by other diagnostic tests, which may be known in the art. The test kit can be used to analyze any kind of sample from an individual including but not limited to whole blood, blood cells (such as leukocytes, B-cells, T-cells, monocytes, macrophages, erythrocytes and thrombocytes), serum, plasma, hemofiltrate, urine, lymph, stool, tissue from subcutaneous fat, from visceral fat, or from other kind of fat, from muscle, form pancreas, form liver, from the small or large intestine or from other tissues and organs as well as combinations thereof. The test can be done with pooled samples of one or more than one individual. The sample can be used directly, or after storage of the sample for various times at various storage condition, or the sample can be a pre-processed, for example the sample can be fractionated by chromatography, filtration, precipitation, liquid or other extraction methods, immunoprecipitation, etc. The whole sample or individual or two or more combined fractions of a pre-processed sample, originating from one or more than one samples can be analyzed. The test can be done once or several times, preferably several times over a period of time to analyze the time course of quantity changes of a peptide, preferably of serum albumin peptides of the invention, preferably of serum albumin peptides with a C-terminal alpha-amidation, preferably of the serum albumin peptides according to Seq.-ID 1 to 5 and of their corresponding nucleic acids, and of fragments or modifications of said peptides and nucleic acids.

The test can be done with any type of test kit known in the art including but not limited to the methods to analyze a sample, as described elsewhere in this patent application.

The test kit can comprise substances such as the serum albumin peptides of the invention, preferably serum albumin peptides with a C-terminal alpha-amidation, preferably serum albumin peptides according to Seq.-ID 1 to 5. Furthermore the test kit can comprise fragments and/or modifications of the said for use as standard and/or for use as controls.

Furthermore the test kit can comprise binding agents for the said, preferably antibodies, preferably antibodies binding specifically to serum albumin peptides comprising a C-terminal alpha-amidation and/or modifications of the said. Furthermore the test kit can comprise nucleic acids hybridizing specifically to the nucleic acids corresponding to said serum albumin peptides.

The binding agents can be present in the test kit in immobilized form, for example immobilized to membranes, to microtiter plates, to mass spectrometric targets, to SELDI chips, to protein chips, to nucleic acid chips, to the surface of microtiter plates, to chromatographic resins, to magnetic particles, to metal particles, to agarose particles, or to other polymer particles, etc. Alternatively or in addition the binding agents can be present in the test kits in a form facilitation their immobilisation to surfaces and materials as noted above. Furthermore the binding agents may be present in labelled form, for example labelled with enzymes, fluorescent dyes, fluorescent proteins or protein fragments or peptides, with different isotopes which can be used in mass spectrometry and/or for radioactive measurements, with dyes measurable by chemiluminescence, photometry or by other measurement methods, with organic or inorganic groups such as biotin, chitin, sugars etc. with lectins, with ligands for receptors or with ligands for other structures, etc.

Furthermore the test kit of the invention can comprise instructions how to use the test kit, how to prepare the samples, what kind of samples to use, how to analyze and interpret the results, etc. Especially the test kit can comprise instructions, how to use the kit for determining the presence or absence or prognosis of disorders selected from the list comprising insulin resistance, glucose intolerance, hyperglycaemia, Type 2 diabetes, preeclampsia, trauma, coagulation disorders and sepsis, deprivation of anti oxidants, copper, iron, cobalt, or nickel or instructions how to use the test kit to predict and/or monitor the success of a therapy for said disorder or to monitor aging, or to monitor narcosis during surgery, or to detect the use of performance-enhancing substances by an athlete, or to detect the application of performance-enhancing substances to animals used in sports.

### Pre-form of type 2 diabetes

With pre-forms of diabetes are meant all kind of disease or conditions, which are believed to result in type 2 diabetes such as impaired glucose tolerance (IGT), impaired fasting glucose, insulin resistance, etc.

### Negative control sample

A negative control sample is a sample of an individual of the same species, which individual is not suffering from a pre-form of type 2 diabetes or from type 2 diabetes. Preferably the negative sample is of the same type, as the sample, for example the sample and the negative sample are both plasma samples or are both urine samples collected in the morning, or are both fat tissue samples collected by biopsy, etc.

### Reference value

A reference value is a known value of the same marker or peptide to be determined in the diagnostic method. The reference value can be determined prior, simultaneous with, or after the value of the sample has been determined.

### Sample

A sample according to the invention is biological material, which has been obtained from an individual, such as whole blood, plasma, serum, hemofiltrate, urine, fat tissue, liver tissue. A sample can also be material indirectly obtained from an individual, such as cells obtained from the individual, which have been cultured in vitro, prior to obtain a sample from these in vitro cultured cells, which can be the cells itself or the cell culture supernatand obtained from these cells. A sample can also be pre-treated prior to analysis with the methods of the invention. Such pre-treatments for example can be storage of the sample at various temperatures such as room temperature, 4°C, 0°C, -20°C, -70°C, -80°C, or at other temperatures, or storage on water ice, or dry ice, or storage in liquid nitrogen or storage in other solid, liquid or gas media. Further pre-treatments among others are filtration of the sample such as ultrafiltration, preferably with molecular cut off value of 1, 3, 5, 30, 50, 100, 150, 300 or 1000 kDa, precipitation of the sample using salts, organic solvents such as ethanol or other alcohols, acetone, etc., separation of the sample into sub-fractions using methods such as chromatography, liquid phase extraction, solid phase extraction, immune precipitation using antibodies, antibody fragments or other substances binding to constituents of the sample. Chromatography methods among others are size exclusion chromatography, anion or cation chromatography, affinity chromatography, capillary chromatography, etc., preferably reverse phase chromatography. If the sample is pre-treated by separation into sub-fractions individual, some or all fractions and/or the flow through and/or any material left on the chromatography media may be used for further analysis of the sample.

### Amount

The expression amount is meant to describe the absolute amount of a peptide or the amount relative of a peptide relative to for example the same peptide in a negative sample or relative to the reference value of the same peptide. Relative means, that not distinct amounts such as mol or mg/lieter etc. are stated, but that for example is stated that the sample contains more, less or the same amount of a certain peptide, as compared to a reference sample, or reference value. The term "more, less or the same amount" in this situation includes also, if only measurement units are stated, such as absorption value, extinctions coefficients, mass spectrometric signal intensities, densitometric measurements of western blots, or other types of measurement values, which do not translate into absolute amounts of the peptide.

### Decreases or absent

With decreased according to the invention is meant, that a peptide is present in a sample in smaller quantities, or amounts, or concentrations, as compared to another sample, or as compared to a control sample, or as compared to a negative control sample, or as compared to a reference value, regardless if these measurements are relative or absolute measurements. With absent according to the invention is meant, that a peptide is not present at all in sample, or that a peptide is present in a quantity, or amount, or concentration in a sample, which quantity, or amount, or concentration is below the detection limit of the test system used to detect, or measure said peptide. Preferably a peptide, which is decreased is present in a concentration, or quantity or amount, which is at least 10%, at least 20%, at least 30%, at least 40%, at least 50%, at least 60%, at least 70%, at least 80%, at least 90, at least 100%, at least 150%, at least 200%, at least 300%, at least 400%, at least 500%, at least 1000%, or at least more than 1000% below the value to which it is compared.

### Increased or present

With increased according to the invention is meant, that a peptide is present in a sample in higher quantities, or amounts, or concentrations, as compared to another sample, or as compared to a control sample, or as compared to a negative control sample, or as compared to a reference value, regardless if these measurements are relative or absolute measurements. Preferably a peptide, which is increased is present in a concentration, or quantity or amount, which is at least 10%, at least 20%, at least 30%, at least 40%, at least 50%, at least 60%, at least 70%, at least 80%, at least 90, at least 100%, at least 150%, at least 200%, at least 300%, at least 400%, at least 500%, at least 1000%, or at least more than 1000% above the value to which it is compared.

### EXAMPLES

### Example 1: Oral glucose tolerance test

8 healthy individuals, 2 patients with impaired glucose tolerance and 3 diabetic patients were enrolled into this study after they provided written informed consent and the local Ethics Committee at the Hannover Medical School approved the protocol. Blood samples were obtained from each individual of the healthy and of the glucose tolerance impaired individuals before and after oral glucose tolerance test as described in example 2. From the diabetic individuals only blood samples after over night fastening were obtained, as an oral glucose tolerance test is contraindicated for diabetic patients. For those individuals subjected to the oral glucose tolerance test the individuals were fastening over night and on the morning orally ingested a solution of 75 g of glucose dissolved in 300 mL of water. Directly prior to ingestion of the glucose, 1 h and 2 h after the ingestion of the glucose solution a blood sample was drawn from each individual. The experiment resulted in three blood samples from each individual of the healthy 8 individuals and of the 2 individuals with an impaired glucose tolerance test and resulting in one blood sample form each of the 3 diabetic individuals. So in total there were in a total of 30 blood samples used for further analysis.

### Example 2: Plasma preparation

Blood samples used to prepare plasma were collected from the cubital vein into 9 ml S-Monovette blood collection tubes containing potassium-EDTA as anticoagulant (Sarstedt, Numbrecht, Germany). Within 30 min. after blood withdrawal, plasma was prepared. The collection tubes were centrifuged for 10 min. at 2000 g at room temperature to remove blood cells from the plasma. To remove residual blood cells and platelets, aliquots of 2 mL plasma were sterile filtered using a 10 mL syringe equipped with a cellulose acetate filter unit with a 0.2 µm pores size and 5 cm2 filtration area (Sartorius Minisart®, Göttingen, Germany). Samples were stored at-80 °C until further processed for reverse phase chromatography or for determination of insulin and C-peptide by enzyme-linked immuno sorbent assay (ELISA).

### Example 3: Determination of insulin and C-peptide by ELISA

Determination of the level of glucose, C-peptide (a fragment of pre-insulin released during processing of pre-insulin to mature insulin), insulin, pre-insulin and of triglycerides was done by the "Medizinisches Labor Hannover", Hannover, Germany according to standard methods known in the art using commercially available enzyme-linked immuno sorbent assays and other assays according to the manufacturer's instructions using small aliquots of the plasma samples stored at -80 °C.

**Table 1: Metadata of the samples**

| Meta data | Patient | 0h | 0h | 1h | 1 h | 2h | 2h |
|---|---|---|---|---|---|---|---|
| | | mean | SD | mean | SD | mean | SD |
| glucose | normal | 84.1 | 12.3 | 100 | 29 | 74.8 | 16.1 |
| glucose | IGT | 133 | 18.4 | 255.5 | 13.4 | 212.5 | 53 |
| glucose | diabetic | 141.7 | 12.5 | | | | |
| C-peptide | normal | 2.2 | 0.9 | 6.7 | 1.4 | 5 | 1.9 |
| C-peptide | IGT | 2.9 | 0.3 | 7.1 | 1.8 | 7.1 | 1.5 |
| C-peptide | diabetic | 3.4 | 2.6 | | | | |
| insulin | normal | 21.5 | 20.9 | 68.9 | 45.3 | 24.6 | 15.4 |
| insulin | IGT | 17.8 | 1.3 | 87.6 | 24.7 | 76 | 39.7 |
| insulin | diabetic | 24.9 | 4.6 | | | | |
| pro-insulin | normal | 55.9 | 150.9 | 177.7 | 437.1 | 44.6 | 36.3 |
| pro-insulin | IGT | 16.6 | 3.2 | 46.5 | 4.9 | 53.5 | 3.5 |
| pro-insulin | diabetic | 38.6 | 29.6 | | | | |
| tri-glyceride | normal | 86.6 | 27.3 | 82.5 | 24.2 | 72.7 | 19.2 |
| tri-glycerides | IGT | 114.5 | 21.9 | 122 | 18.4 | 114 | 5.7 |
| tri-glycerides | diabetic | 300 | 176.1 | | | | |

| | | | | | | | |
|---|---|---|---|---|---|---|---|
| IGT = impaired glucose tolerance = perform of diabetes | | | | | | | |

### Example 4: Sample preparation for reverse phase chromatography:

To separate the peptides from high molecular weight proteins the plasma samples were thawed and subjected to ultrafiltration using Amicon ultra centrifugal filter devices with a molecular weight cut off of 50 kDa (Millipore, Bedford, MA, USA). The ultrafiltation units rinsed prior to use and were used according to the manufacturer's instructions. 1.3 mL of plasma was added to 3.9 mL of 8 M guanidiniumchloride in a 15 mL polypropylene tube at 23 °C and immediately vigorously mixed and transferred to the ultrafiltration device. Ultrafiltration took place in a swinging bucket rotor for 60 min. at 4000 g. The filtrate, subsequently termed "peptide extract" was stored at -80 °C until further processed or was directly subjected to reverse phase chromatography.

### Example 5: Liquid chromatography of the samples

Prior to chromatography the samples were centrifuged at 4 °C and 18 000 g for 10 minutes, the pH was adjusted to pH 2 to pH 3 using a stock solution of 30 % (v/v) HCl and the samples were diluted using a 0.05 % (v/v) stock solution of trifluoroacetic acid. Peptide extracts corresponding to 0.75 mL plasma equivalents were processed via the Agilent 1100 chromatography system at constant 33 °C (Agilent Technologies, Böblingen, Germany) using a reverse phase high pressure liquid chromatography (HPLC) column, containing Source 5RPC as matrix, with the dimensions 4.6 x 150 mm (Amersham Biosciences Europe GmbH, Freiburg, Germany). A linear gradient form 4% to 40% acetonitrile within 48 min. at a flow rate of 0.5 mL/min. with collection of 96 fractions, each 0.25 mL in volume. The buffers used for the gradient were Buffer A: 0.06 % volume per volume (v/v) trifluoroacetic acid (TFA) and 99,94 % (v/v) distilled water and buffer B: 0.05 % (v/v) TFA, 80 % (v/v) acetonitrile and 19.95 % (v/v) distilled water.

The standard peptides used were: ACTH 1-17, ACTH 1-24, neurotensin, substance P, big endothelin 1 (19-38), ACTH 18-39, beta-endorphin 6-31, somatostatin-14, renin substrate (porcine), GRF 1-44 (bovine), calcitonin, insulin B chain oxide (bovine), Pro34-NPY (porcine) and they were added to the samples prior to ultrafiltration. Independent to sample volume there were added 500 fmol of each standard peptide into each sample.

### Example 6: Mass spectrometric analysis of the samples

For mass spectrometric analysis, typical positive ion spectra of peptides were measured using a MALDI-TOF (matrix-assisted laser desorption ionization time of flight) mass spectrometer. Suitable MALDI-TOF mass spectrometers among others are manufactured by Applied BioSystems, Framingham, USA (Voyager-DE, Voyager-DE PRO or Voyager-DE STR) or by Bruker Daltonik, Bremen, Germany (BIFLEX). Typical matrix substances suitable for peptides are matrix substances, which comprise an organic acid such as 3,5-dimethoxy-4-hydroxycinnamic acid, alpha-cyano-4-hydroxycinnamic acid and 2,5-dihydroxybenzoic acid. For MALDI sample preparation we used alpha-cyano-4-hydroxycinnamic acid as matrix and 6-desoxy-I-galactose as co-matrix, dissolved in acetonitrile containing 0.1 % TFA. A lyophilized equivalent obtained by reverse phase chromatography corresponding to 15 µL equivalents of the plasma fractions dissolved in 0.3 µL matrix solution were spotted onto the mass spectrometric carrier plate, dried and analyzed in a Voyager-DE STR MALDI mass spectrometer from Applied BioSystems.

Mass spectrometry can be employed to quantify peptides such as, for example, the peptides of the invention if these peptides are present in a concentration which is within the dynamic measurement range of the mass spectrometer, thus avoiding detector saturation. The ability of MALDI mass spectrometry to quantitatively measure individual peptide and/or protein mass signals was confirmed by adding known concentrations of 50 to 800 pmol peptides, such as neurotensin, into complex biological samples such as plasma samples. Subsequently these samples were separated by reversed phase chromatography and those fractions containing the added peptides were analyzed by mass spectrometry as described in the examples. There is a the linear correlation between the MALDI mass spectrometry mass signal intensity of for example the neurotensin peptide and the amount of the neurotensin peptide added to the plasma sample, indicating that MALDI mass spectrometry is suitable to quantitatively determine peptides in complex samples such as plasma.

Figure 3 shows results obtained with a plasma sample of a healthy individual. The sample was intentionally chosen to contain two roughly equally signal intensities for the serum albumin fragment representing amino acids 25 to 51 with and without a C-terminal alpha-amide modification. This figure clearly demonstrates that there exist 2 different forms of this serum albumin peptide. Panel A shows a small section of a so called "peptide map", where the x-axis depicts the molecular mass, the y-axis represents the individual fractions analyzed by mass spectrometry and the colour intensity (in grey scale) depicts the mass spectrometric signal intensity. The albumin peptides elute mainly in two different fractions. The non-modified albumin 25-51 elutes predominantly in fraction 74, whereas the C-terminal alpha-amidated albumin 25-51 (albumin 25-51 amide) elutes predominantly in fraction 76. In some samples fraction 75 contains both albumin 25-51 peptides. Panel B shows an enlarged, very small section of the peptide map shown in panel A. It can be seen, that the albumin 25-51 amide has a slightly lower molecular mass of about 1 Dalton as compared to the albumin 25-51 without an amid modification. The signal of albumin 25-51 is spread over a mass range of several Daltons due to the presence of different isotopes, primary carbon isotopes, present in nature, which also result in a several shifts of 1 Dalton each. Panel C finally shows a high resolution mass spectrum of Fraction 74 and 76 of a plasma sample from a healthy individual. Due to the high resolution of this mass spectrum albumin 25-51 peptides with different natural carbon isotope distributions can be resolved. This results in 5 distinct peaks, which are differing in their molecular masses in increments of 1 Dalton. All 5 distinct albumin 25-51 peaks are shifted in the case of the albumin 25-51 amide to molecular mass which is 1 Dalton lower, as compared to the mass spectrum of Albumin 25-51 without an amid modification. The modification of the C-terminus into an alpha-amide results in a reduction of the molecular mass of about 1 Dalton.

### Example 7: Peptide identification

Detection of peptides present in different quantities or of peptides missing or newly appearing in certain samples was achieved by calculation of subtractive peptide display maps and correlation analysis. Selected peptides were identified by use of either MALDI TOF-TOF (4700 Proteomics Analyzer from Applied BioSystems, Framingham, USA), ESI-qQTOF (QSTAR Pulsar from Applied BioSystems, Framingham, USA) or ESI-qQTOF (QUTOF Ultima from Micromass, Manchester, Great Britain) (MALDI TOF-TOF = Matrix Assisted Laser Desorption lonisation Time Of Flight-Time Of Flight; ESI-qQTOF = Electro Spray lonisation-quatropole Quattropole Time Of Flight). For sequencing with the ESIqQTOF instruments preparative sample preparations were done, using the same reverse phase material, the same buffers and the buffer gradients as used for generation of the data for the peptide maps. Sequencing with the MALDI TOF-TOF was done using either directly the samples used for preparing of the peptide maps, or using preparative sample preparations. Peptide ions were selected in the mass spectrometer on the basis of their specific m/z (mass/charge) values in a manner known to the skilled worker. These selected ions were then fragmented by supplying collision energy with an collision gas, and the resulting fragments of the peptides or proteins were detected in the mass spectrometer in an integrated analysis unit, and corresponding m/z values were determined (principle of tandem mass spectrometry). The fragmentation behaviour of peptides enables unambiguous identification of the peptides. The resulting peptide fragment spectra were detected using nanoSpray in the product ion scan mode. Up to 200 scans per sample were accumulated. Charge state deconvolution was performed using the Bayesian reconstruct tool of the BioAnalyst program package (PE Applied BioSystems), deisotoping was achieved by means of the Voyager 5.1 software (PE Applied BioSystems). The mass spectra were saved in a MASCOT generic file format, and submitted to the MASCOT19 database search engine (Matrix Science, London, Great Britain). Searched databases were SwissProt (www.expasy.ch) and MSDB (EBI, Europe). In addition, peptide sequencing method allows the identification of amino acid modifications such as C-terminal alpha-amidation, even as such a modification only results in a mass difference of 1 Dalton.

Figure 4 shows the mass spectrometric identification of the albumin 25-51 amide, as described in the examples. The albumin 25-51 amide is fragmented in a mass spectrometer and the molecular masses of the resulting fragments are determined. Using sequence databases it was calculated, that the determined mass spectrometric-induced fragments of the proposed albumin 25-51 amide indeed originated from albumin 25-51 amide. This is a standard method used in the art to determine peptide sequences including modifications present in these peptides.

Figure 6 shows an example for the analysis of N-terminal proGIP. Two peptides were indentified both originating from the N-terminal proGIP. These peptides represent aa residues 22 to 51 and aa residues 30 to 51, respectively. In Figure 6 peptide aa 22 to 51 is shown. Both peptides were elevated in samples after glucose ingestion.

### Example 8: Data analysis

Subsequently to fractionation, as described in example 5, each fraction was individually analyzed by mass spectrometry, as described in example 6, resulting in 96 mass spectra for each individual plasma sample. Data were analyzed, including peak recognition and visualization using the in-hose developed software package Spectromania®. Quantification of mass spectrometric signals was performed after baseline correction by integrating absolute signal intensities in 1 Dalton bins.

For easier visualization, the mass spectrum of each fraction was transformed to a virtual one-dimensional gel image. The molecular mass of each peptide is indicated by its position within the virtual gel lane, whereas the mass spectrometric signal intensity for each peptide is indicated by the colour intensity (in grey-scale) of the corresponding bar. Figure 3 and Figure 6, described above, shows such one-dimensional gel images of individual fractions of plasma obtained from human individuals subjected to an oral glucose tolerance test.

To analyze the complete peptide composition of samples the 96 mass spectra of individual plasma samples, transformed into such one-dimensional gel images, are combined, resulting in a two-dimensional display of peptides termed peptide display. The x-axis of such a peptide display depicts the molecular mass (m/z) of the peptides, the y-axis depicts the fraction into which the peptide eluted (possibly a peptide elutes in two or more neighbouring fractions), and the quantity of the peptide, e.g. it's mass spectrometric signal intensity, is depicted by the colour intensity (shown in grey-scale). Such peptide maps, obtained from the same group of sample, e.g. plasma sample collected before the oral glucose tolerance test or plasma samples collected after the oral glucose tolerance test were combined and a corresponding mean peptide map of each group was calculated. Prior to calculating these mean maps the individual peptide maps were normalized (adjustment for potential minor fraction and mass shifts by use of the known positions within the peptide maps (fraction and m/z = mass) oft the added 14 peptide standards.

Detection of differently expressed peptides was achieved by calculation of differential peptide displays or correlation analysis. Methods used for correlation analysis are described in detail in EP1533515A1. Differential peptide displays can be obtained by either electronically subtracting the signal intensities of individual peptide maps of individual plasma sample from the same human individual before and after the oral glucose tolerance test or by subtracting the signal intensities of mean maps of plasma samples obtained before oral glucose tolerance test from the signal intensities of mean maps of plasma samples obtained after oral glucose tolerance test. The data of peptide displays may be pre-processed by adjusting for background noise and outliers may be removed from the analysis. For correlation analysis each mass signal intensity above a threshold was tested for correlation to any other mass signal intensity above the same certain threshold of mass signal intensity. Correlation analysis identifies signals which either behave the same (e.g. both signals go up or go down) or which behave opposite (e.g. one signal goes up, whereas the other signal goes down). Correlation analysis was also performed using additional meta data such as plasma insulin or plasma c-peptide level. The c-peptide is a distinct fragment released from the insulin precursor during it's processing to form active insulin. Mean mass maps of both groups served for the purpose of comparison between groups and as a template for the definition of peak coordinates. For this purpose, a threshold level was set to clearly distinguish signals from baseline noise. Signal intensities of all peaks that fulfilled threshold criteria were exported for comprehensive correlation data analysis with commercially available software.

During the analysis it was observed, that the samples of 2 of the healthy individuals resulted in inconsistent data as compared to the other 6 healthy control individuals and consequently were omitted from the subsequent analysis.

From the data obtained in the above described Examples it is clear that e.g. after induction of oxidative stress - ingestion of glucose - healty individuals and IGT patients can be distinguished in view of the altered expression of C-terminal alpha amidation of serum albumin fragments or of the N-terminal or C-terminal proGIP. In particular, it can be derived from the examples that in IGT patients lower amounts of C-terminal alpha-amidated serum albumin peptides can be detected. In particular, the increment after glucose ingestion to initiate oxidative stress demonstrates that the amount of amidated serum albumin peptide is reduced in comparison to healthy individuals. Thus, a reduced amount of C-terminal alpha amidated serum albumin fragment is indicative for reduced oxidative stress capacity in an individual. In addition, since many of the complications related to diabetes or other diseases and conditions are explained by oxidative stress, the determination of C-terminal alpha amidated peptides represents a means for detecting and diagnosing said diseases and conditions. From Figure 2 it can be derived that only the determination of the amidated peptide allows for the determination of oxidative capacity of an individual. The amount of the peptide not having the C-terminal alpha-amid modification remains basically unchanged. Furthermore, figure 2 demonstrates that healthy individuals have a stronger increase after induction of oxidative stress than individual suffering from IGT.

With respect to N-terminal and C-terminal proGIP it was demonstrated that said peptides are elevated after oGTT and that the amount of said peptides remain high even after 2 hours in individuals having IGT and diabetes in contrast to normal individuals showing a decrease of said peptides after two hours compared to the amount detectable one hour after glucose ingestion.

## Claims

1. A method for the diagnosis of an individual for the presence of a pre-form of type 2 diabetes, such as impaired glucose tolerance (IGT) comprising the steps of:
a. determining the relative or absolute amount of a at least one peptide selected from the group consisting of (i) Seq-ID No. 19 to 21 or a modified form thereof or (ii) at least one peptide comprising a C-terminal alpha-amidation, said amidated peptide is derived from a precursor peptide or protein and at least the C-terminal amino acid of said precursor peptide or protein is absent in said amidated peptide, whereby the alpha-amidation can not be originated from enzymatic cleavage and amidation in a sample of an individual;
b. comparing the result of a) with the result determined using a negative control sample or with an already know reference value; and
determining the presence or absence of a pre-form of type 2 diabetes, such as IGT, in said individual.

2. A method for detecting oxidative stress in an individual or a sample of said individual by determining the relative or absolute amount of at least one peptide comprising a C-terminal alpha-amidation, said amidated peptide is derived from a precursor peptide or protein and at least the C-terminal amino acid of said precursor peptide or protein is absent in said amidated peptide, whereby the alpha-amidation can not be originated from enzymatic cleavage and amidation, the method comprising the step of:
a. measuring the amount of said at least one peptide in an individual or a sample of said individual and
b. comparing the determined amount with a reference amount of said at least one peptide.

3. The method according to claim 1 or 2, wherein said precursor peptide or protein, from which said amidated peptide originates,
A.)
a. comprises a binding site for transient metal ion, e.g. copper, cobalt, nickel or iron ions, and/or
b. comprises a free sulfhydryl group, and/or
c. comprises a nitric oxide binding site, and
B.) does not have a glycine residue at the amino acid position following the amino acid residue which is C-terminally amidated in the amidated peptide.

4. The method according to any one of claims 1 to 3, wherein said at least one peptide is a fragment of serum albumin.

5. The method according to claim 4, wherein the serum albumin is human serum albumin having the sequence shown in Seq.-ID 16 or naturally occurring mutants thereof.

6. The method according to any one of claims 1 to 5, wherein said at least one peptide is selected from Seq.-ID 1 to 5.

7. A method for detecting and/or diagnosing a disease or condition selected from insulin resistance, impaired glucose intolerance, impaired fasting glucose, glucose intolerance, hyperglycaemia, Type 2 diabetes, ischemia, stroke, hypoxemia, preeclampsia, trauma, coagulation disorders, sepsis, and deprivation of anti oxidants, copper, iron, cobalt and/or nickel by determining the relative or absolute amount of at least one peptide comprising a C-terminal alpha-amidation, said amidated peptide is derived from a precursor peptide or protein and at least the C-terminal amino acid of said precursor peptide or protein is absent in said amidated peptide, whereby the alpha-amidation can not be originated from enzymatic cleavage and amidation, the method comprising the step of:
a. measuring the amount of said at least one peptide in an individual or a sample of said individual and
b. comparing the result of a) with the result determined using a negative control sample or with an already known reference value, and determining the presence or absence of said disease or condition in said individual.

8. A method for monitoring the therapy of a condition or disease selected from insulin resistance, impaired glucose tolerance, impaired fasting glucose, glucose intolerance, hyperglycaemia, Type 2 diabetes, ischemia, stroke, hypoxemia, preeclampsia, trauma, coagulation disorders, sepsis, and deprivation of anti oxidants, copper, iron, cobalt or nickel or for monitoring of aging, for monitoring of narcosis during surgery, or to detect the use of performance-enhancing substances by an athlete or to detect the application of performance-enhancing substances to animals used in sports by determining the relative or absolute amount of a peptide selected from the group consisting of (i) Seq-ID No. 19 to 21 or a modified form thereof or (ii) at least one peptide comprising a C-terminal alpha-amidation, said amidated peptide is derived from a precursor peptide or protein and at least the C-terminal amino acid of said precursor peptide or protein is absent in said amidated peptide, whereby the alpha-amidation can not be originated from enzymatic cleavage and amidation, the method comprising the step of of:
a. measuring the amount of said at least one peptide in an individual or a sample of said individual and
b. comparing the result of a) with the result determined amountusing a negative control sample or with aan already know reference amount of said at least one peptide.value.

9. The method according to any one of the preceding claims wherein the at least one peptide is a peptide of Seq-ID No. 19 or 20 or a modified form thereof.

10. The method according to any one of claims 1 to 9 wherein said determination of said at least one peptide is effected by a method selected from ELISA, RIA, Western blotting, FACS analysis, plasmon resonance, immuno precipitation, and mass spectrometry.

11. The method according to any one of the preceding claims, wherein said sample is selected from the group consisting of whole blood, plasma, serum, urine, fat tissue and liver tissue.

12. A peptide derived from serum albumin of Seq.-ID 16 comprising the N-terminal sequence Asp-Ala-His-Lys-Xaa (Seq.-ID 17), wherein Xaa represents amino acid residue(s) of between 0 and 23 consecutive amino acid residues of amino acids 5 to 27 according to Seq.-ID 16 starting with amino acid at position 5 of Seq.-ID 16, said peptide comprises an alpha-amide modification of the C-terminal amino acid residue of said peptide.

13. The peptide according to claim 12 having an amino acid sequence according to Seq.-ID 1 to 5.

14. The peptide according to claim 12 or 13 comprising at least one transition metal ion bound by coordination to said peptide.

15. The peptide according to claim 14 wherein said transition metal ion is selected from the group comprising of copper ions, cobalt ions, nickel ions and iron ions.

16. A serum albumin peptide originating from Seq.-ID 16 without the amino acid residues of Seq.-ID 17 resulting from cleaving off the peptide of Seq.-ID 17.

17. A serum albumin peptide originating form Seq.-ID 16 resulting from cleaving off of another peptide of Seq.-ID 17 said albumin peptide having at its N-terminus a carbonyl group.

18. The serum albumin peptide originating form Seq.-ID 16 and resulting from cleaving off of a peptide according to any one of Seq.-ID 1 to 5.

19. The peptide having the amino acid sequence of Seq-ID No. 20 or modifications thereof.

20. A modification of a peptide according to any one of claims 12 to18, which mutant shows at least 70 % sequence identity to the corresponding non-modified sequence as depicted in Seq.-ID 16.

21. A binding agent, specific for a protein or peptide having a C-terminal alpha-amide modification which can distinguish between said peptide having a C-terminal alpha-amide and the same peptide not having said C-terminal alpha-amide.

22. A binding agent, specific for a peptide according to claim 12 or 14.

23. A binding agent according to claim 21 or 22, which is an antibody or fragment or modification thereof, which antibody or fragment or modification thereof retains its binding specificity for its epitope.

24. A test kit for performing a method according to any one of claims 1 to 11 comprising means for detecting in said individual or a sample therefrom the amount of the at least one peptide as defined in any of claims 1 to 11.

25. A test kit according to claim 24, which comprises a binding agent according to claims 21 or 22 or which is specific for a peptide of Seq-ID. No. 19 to 21 or a modification thereof and/or a standard which comprises a peptide according to any one of claims 12 to 20, and instructions how to use said kit.

26. A method for the manufacture of a peptide according to any one of claims 12 to 18 or according to claim 20, comprising the step of contacting serum albumin with metal ions of a transition metal and/or, reducing agents, and/or radicals, and/or nitric oxide, resulting in nonenzymatic cleavage of serum albumin and/or loading of serum albumin fragments with nitric oxide and/or transition metal ions.

27. A composition comprising a peptide according to any one of claims 12 to20, and/or a binding agent according to any one of claims 21 or 22 for medical uses.

28. A composition comprising a peptide according to any one of claims 16 to 18 or according to claim 20 for medical use as an in vivo nitric oxide delivery vehicle.

29. A method for controlling the presence or absence of a serum albumin peptide according to any one of claim 12 to 18 or according to claim 20 for stratification of a therapeutical regime to treat a condition selected from the list comprising of insulin resistance, impaired glucose tolerance, impaired fasting glucose, glucose intolerance, hyperglycaemia, Type 2 diabetes, ischemia, stroke, hypoxemia, preeclampsia, trauma, coagulation disorders and sepsis, and deprivation of anti oxidants, copper, iron, cobalt or nickel.

30. A method for diagnosis of insulin resistance, impaired glucose tolerance, impaired fasting glucose, glucose intolerance, hyperglycaemia, Type 2 diabetes, preeclampsia, trauma, coagulation disorders and sepsis, deprivation of anti oxidants, copper, iron, cobalt, or nickel, or for monitoring of aging, for monitoring of narcosis during surgery, or to detect the use of performance-enhancing substances by an athlete or to detect the application of performance-enhancing substances to animals used in sports by use of the Ischemia Modified Albumin assay.

31. A method for diagnosis of insulin resistance, impaired glucose tolerance, impaired fasting glucose, glucose intolerance, hyperglycaemia, Type 2 diabetes, preeclampsia, deprivation of anti oxidants, deprivation of copper, iron, cobalt or nickel ions, trauma, coagulation disorders and sepsis or for monitoring of aging, for monitoring of narcosis during surgery, or to detect the use of performance-enhancing substances by an athlete or to detect the application of performance-enhancing substances to animals used in sports, by measurement of the binding of copper, cobalt, nickel, or iron ions to serum albumin in a sample from an organism.

32. Use of a method according to any one of claims 1 to 11 for diagnostic purposes in combination with an Ischemia Modified Albumin assay and/or an assay detecting Haemoglobin A1c (HbA1c) to improve the specificity and/or selectivity of the diagnosis.


SEQUENCE LISTING

<110>  BioVisioN AG

<120>  Medical uses for non-enzymatic originated C-terminal alpha amides
        of peptides

<130>  Albu-Amid

<160>  23

<170>  PatentIn version 3.3

<210>  1
<211>  27
<212>  PRT
<213>  Homo sapiens


<220>
<221>  MOD_RES
<222>  (27)..(27)
<223>  AMIDATION

<400>  1

Asp Ala His Lys Ser Glu Val Ala His Arg Phe Lys Asp Leu Gly Glu
1               5                   10                  15


Glu Asn Phe Lys Ala Leu Val Leu Ile Ala Phe
            20                  25


<210>  2
<211>  26
<212>  PRT
<213>  Homo sapiens


<220>
<221>  MOD_RES
<222>  (26)..(26)
<223>  AMIDATION

<400>  2

Asp Ala His Lys Ser Glu Val Ala His Arg Phe Lys Asp Leu Gly Glu
1               5                   10                  15


Glu Asn Phe Lys Ala Leu Val Leu Ile Ala
            20                  25


<210>  3
<211>  24
<212>  PRT
<213>  Homo sapiens


<220>
<221>  MOD_RES
<222>  (24)..(24)
<223>  AMIDATION

<400>  3

Asp Ala His Lys Ser Glu Val Ala His Arg Phe Lys Asp Leu Gly Glu
1               5                   10                  15

Glu Asn Phe Lys Ala Leu Val Leu
            20

<210>  4
<211>  23
<212>  PRT
<213>  Homo sapiens

<220>
<221>  MOD_RES
<222>  (23)..(23)
<223>  AMIDATION

<400>  4

Asp Ala His Lys Ser Glu Val Ala His Arg Phe Lys Asp Leu Gly Glu
1               5                   10                  15

Glu Asn Phe Lys Ala Leu Val
            20

<210>  5
<211>  13
<212>  PRT
<213>  Homo sapiens

<220>
<221>  MOD_RES
<222>  (13)..(13)
<223>  AMIDATION

<400>  5

Asp Ala His Lys Ser Glu Val Ala His Arg Phe Lys Asp
1               5                   10

<210>  6
<211>  27
<212>  PRT
<213>  Homo sapiens

<400>  6

Asp Ala His Lys Ser Glu Val Ala His Arg Phe Lys Asp Leu Gly Glu
1               5                   10                  15

Glu Asn Phe Lys Ala Leu Val Leu Ile Ala Phe
            20                  25

<210>  7
<211>  26
<212>  PRT
<213>  Homo sapiens

<400>  7

Asp Ala His Lys Ser Glu Val Ala His Arg Phe Lys Asp Leu Gly Glu
1               5                   10                  15

Glu Asn Phe Lys Ala Leu Val Leu Ile Ala
            20                  25

<210>  8
<211>  24
<212>  PRT
<213>  Homo sapiens

<400>  8

Asp Ala His Lys Ser Glu Val Ala His Arg Phe Lys Asp Leu Gly Glu
1               5                   10                  15

Glu Asn Phe Lys Ala Leu Val Leu
            20

<210>  9
<211>  23
<212>  PRT
<213>  Homo sapiens

<400>  9

Asp Ala His Lys Ser Glu Val Ala His Arg Phe Lys Asp Leu Gly Glu
1               5                   10                  15

Glu Asn Phe Lys Ala Leu Val
            20

<210>  10
<211>  13
<212>  PRT
<213>  Homo sapiens

<400>  10

Asp Ala His Lys Ser Glu Val Ala His Arg Phe Lys Asp
1               5                   10

<210>  11
<211>  576
<212>  PRT
<213>  Homo sapiens

<400>  11

Ala Gln Tyr Leu Gln Gln Cys Pro Phe Glu Asp His Val Lys Leu Val
1               5                   10                  15

Asn Glu Val Thr Glu Phe Ala Lys Thr Cys Val Ala Asp Glu Ser Ala
            20                  25                  30

Glu Asn Cys Asp Lys Ser Leu His Thr Leu Phe Gly Asp Lys Leu Cys
        35                  40                  45

Thr Val Ala Thr Leu Arg Glu Thr Tyr Gly Glu Met Ala Asp Cys Cys
    50                  55                  60

Ala Lys Gln Glu Pro Glu Arg Asn Glu Cys Phe Leu Gln His Lys Asp
65                  70                  75                  80

Asp Asn Pro Asn Leu Pro Arg Leu Val Arg Pro Glu Val Asp Val Met
                85                  90                  95

Cys Thr Ala Phe His Asp Asn Glu Glu Thr Phe Leu Lys Lys Tyr Leu
            100                 105                 110

Tyr Glu Ile Ala Arg Arg His Pro Tyr Phe Tyr Ala Pro Glu Leu Leu
        115                 120                 125

Phe Phe Ala Lys Arg Tyr Lys Ala Ala Phe Thr Glu Cys Cys Gln Ala
    130                 135                 140

Ala Asp Lys Ala Ala Cys Leu Leu Pro Lys Leu Asp Glu Leu Arg Asp
145                 150                 155                 160

Glu Gly Lys Ala Ser Ser Ala Lys Gln Arg Leu Lys Cys Ala Ser Leu
                165                 170                 175

Gln Lys Phe Gly Glu Arg Ala Phe Lys Ala Trp Ala Val Ala Arg Leu
            180                 185                 190

Ser Gln Arg Phe Pro Lys Ala Glu Phe Ala Glu Val Ser Lys Leu Val
        195                 200                 205

Thr Asp Leu Thr Lys Val His Thr Glu Cys Cys His Gly Asp Leu Leu
    210                 215                 220

Glu Cys Ala Asp Asp Arg Ala Asp Leu Ala Lys Tyr Ile Cys Glu Asn
225                 230                 235                 240

Gln Asp Ser Ile Ser Ser Lys Leu Lys Glu Cys Cys Glu Lys Pro Leu
                245                 250                 255

Leu Glu Lys Ser His Cys Ile Ala Glu Val Glu Asn Asp Glu Met Pro
            260                 265                 270

Ala Asp Leu Pro Ser Leu Ala Ala Asp Phe Val Glu Ser Lys Asp Val
        275                 280                 285

Cys Lys Asn Tyr Ala Glu Ala Lys Asp Val Phe Leu Gly Met Phe Leu
    290                 295                 300

Tyr Glu Tyr Ala Arg Arg His Pro Asp Tyr Ser Val Val Leu Leu Leu
305                 310                 315                 320

Arg Leu Ala Lys Thr Tyr Glu Thr Thr Leu Glu Lys Cys Cys Ala Ala
                325                 330                 335

Ala Asp Pro His Glu Cys Tyr Ala Lys Val Phe Asp Glu Phe Lys Pro
            340                 345                 350

Leu Val Glu Glu Pro Gln Asn Leu Ile Lys Gln Asn Cys Glu Leu Phe
        355                 360                 365

Glu Gln Leu Gly Glu Tyr Lys Phe Gln Asn Ala Leu Leu Val Arg Tyr
    370                 375                 380

Thr Lys Lys Val Pro Gln Val Ser Thr Pro Thr Leu Val Glu Val Ser
385                 390                 395                 400

Arg Asn Leu Gly Lys Val Gly Ser Lys Cys Cys Lys His Pro Glu Ala
                405                 410                 415

Lys Arg Met Pro Cys Ala Glu Asp Tyr Leu Ser Val Val Leu Asn Gln
            420                 425                 430

Leu Cys Val Leu His Glu Lys Thr Pro Val Ser Asp Arg Val Thr Lys
        435                 440                 445

Cys Cys Thr Glu Ser Leu Val Asn Arg Arg Pro Cys Phe Ser Ala Leu
    450                 455                 460

Glu Val Asp Glu Thr Tyr Val Pro Lys Glu Phe Asn Ala Glu Thr Phe
465                 470                 475                 480

Thr Phe His Ala Asp Ile Cys Thr Leu Ser Glu Lys Glu Arg Gln Ile
                485                 490                 495

Lys Lys Gln Thr Ala Leu Val Glu Leu Val Lys His Lys Pro Lys Ala
            500                 505                 510

Thr Lys Glu Gln Leu Lys Ala Val Met Asp Asp Phe Ala Ala Phe Val
        515                 520                 525

Glu Lys Cys Cys Lys Ala Asp Asp Lys Glu Thr Cys Phe Ala Glu Glu
    530                 535                 540

Gly Lys Lys Thr Cys Cys Cys Lys Ser Ser Cys Leu Arg Leu Ile Thr
545                 550                 555                 560

Ser His Leu Lys Ala Ser Gln Pro Thr Met Arg Ile Arg Glu Arg Lys
                565                 570                 575

<210>  12
<211>  577
<212>  PRT
<213>  Homo sapiens

<400> 12

Phe Ala Gln Tyr Leu Gln Gln Cys Pro Phe Glu Asp His Val Lys Leu
1               5                   10                  15

Val Asn Glu Val Thr Glu Phe Ala Lys Thr Cys Val Ala Asp Glu Ser
            20                  25                  30

Ala Glu Asn Cys Asp Lys Ser Leu His Thr Leu Phe Gly Asp Lys Leu
        35                  40                  45

Cys Thr Val Ala Thr Leu Arg Glu Thr Tyr Gly Glu Met Ala Asp Cys
    50                  55                  60

Cys Ala Lys Gln Glu Pro Glu Arg Asn Glu Cys Phe Leu Gln His Lys
65                  70                  75                  80

Asp Asp Asn Pro Asn Leu Pro Arg Leu Val Arg Pro Glu Val Asp Val
                85                  90                  95

Met Cys Thr Ala Phe His Asp Asn Glu Glu Thr Phe Leu Lys Lys Tyr
            100                 105                 110

Leu Tyr Glu Ile Ala Arg Arg His Pro Tyr Phe Tyr Ala Pro Glu Leu
        115                 120                 125

Leu Phe Phe Ala Lys Arg Tyr Lys Ala Ala Phe Thr Glu Cys Cys Gln
    130                 135                 140

Ala Ala Asp Lys Ala Ala Cys Leu Leu Pro Lys Leu Asp Glu Leu Arg
145                 150                 155                 160

Asp Glu Gly Lys Ala Ser Ser Ala Lys Gln Arg Leu Lys Cys Ala Ser
                165                 170                 175

Leu Gln Lys Phe Gly Glu Arg Ala Phe Lys Ala Trp Ala Val Ala Arg
            180                 185                 190

Leu Ser Gln Arg Phe Pro Lys Ala Glu Phe Ala Glu Val Ser Lys Leu
        195                 200                 205

Val Thr Asp Leu Thr Lys Val His Thr Glu Cys Cys His Gly Asp Leu
    210                 215                 220

Leu Glu Cys Ala Asp Asp Arg Ala Asp Leu Ala Lys Tyr Ile Cys Glu
225                 230                 235                 240

Asn Gln Asp Ser Ile Ser Ser Lys Leu Lys Glu Cys Cys Glu Lys Pro
                245                 250                 255

Leu Leu Glu Lys Ser His Cys Ile Ala Glu Val Glu Asn Asp Glu Met

            260                 265                 270

Pro Ala Asp Leu Pro Ser Leu Ala Ala Asp Phe Val Glu Ser Lys Asp
        275                 280                 285

Val Cys Lys Asn Tyr Ala Glu Ala Lys Asp Val Phe Leu Gly Met Phe
    290                 295                 300

Leu Tyr Glu Tyr Ala Arg Arg His Pro Asp Tyr Ser Val Val Leu Leu
305                 310                 315                 320

Leu Arg Leu Ala Lys Thr Tyr Glu Thr Thr Leu Glu Lys Cys Cys Ala
                325                 330                 335

Ala Ala Asp Pro His Glu Cys Tyr Ala Lys Val Phe Asp Glu Phe Lys
            340                 345                 350

Pro Leu Val Glu Glu Pro Gln Asn Leu Ile Lys Gln Asn Cys Glu Leu
        355                 360                 365

Phe Glu Gln Leu Gly Glu Tyr Lys Phe Gln Asn Ala Leu Leu Val Arg
    370                 375                 380

Tyr Thr Lys Lys Val Pro Gln Val Ser Thr Pro Thr Leu Val Glu Val
385                 390                 395                 400

Ser Arg Asn Leu Gly Lys Val Gly Ser Lys Cys Cys Lys His Pro Glu
                405                 410                 415

Ala Lys Arg Met Pro Cys Ala Glu Asp Tyr Leu Ser Val Val Leu Asn
            420                 425                 430

Gln Leu Cys Val Leu His Glu Lys Thr Pro Val Ser Asp Arg Val Thr
        435                 440                 445

Lys Cys Cys Thr Glu Ser Leu Val Asn Arg Arg Pro Cys Phe Ser Ala
    450                 455                 460

Leu Glu Val Asp Glu Thr Tyr Val Pro Lys Glu Phe Asn Ala Glu Thr
465                 470                 475                 480

Phe Thr Phe His Ala Asp Ile Cys Thr Leu Ser Glu Lys Glu Arg Gln
                485                 490                 495

Ile Lys Lys Gln Thr Ala Leu Val Glu Leu Val Lys His Lys Pro Lys
            500                 505                 510

Ala Thr Lys Glu Gln Leu Lys Ala Val Met Asp Asp Phe Ala Ala Phe
        515                 520                 525

Val Glu Lys Cys Cys Lys Ala Asp Asp Lys Glu Thr Cys Phe Ala Glu

530                 535                 540

Glu Gly Lys Lys Thr Cys Cys Cys Lys Ser Ser Cys Leu Arg Leu Ile
545                 550                 555                 560

Thr Ser His Leu Lys Ala Ser Gln Pro Thr Met Arg Ile Arg Glu Arg
                565                 570                 575

Lys

<210>  13
<211>  579
<212>  PRT
<213>  Homo sapiens

<400>  13

Ile Ala Phe Ala Gln Tyr Leu Gln Gln Cys Pro Phe Glu Asp His Val
1               5                   10                  15

Lys Leu Val Asn Glu Val Thr Glu Phe Ala Lys Thr Cys Val Ala Asp
            20                  25                  30

Glu Ser Ala Glu Asn Cys Asp Lys Ser Leu His Thr Leu Phe Gly Asp
        35                  40                  45

Lys Leu Cys Thr Val Ala Thr Leu Arg Glu Thr Tyr Gly Glu Met Ala
    50                  55                  60

Asp Cys Cys Ala Lys Gln Glu Pro Glu Arg Asn Glu Cys Phe Leu Gln
65                  70                  75                  80

His Lys Asp Asp Asn Pro Asn Leu Pro Arg Leu Val Arg Pro Glu Val
                85                  90                  95

Asp Val Met Cys Thr Ala Phe His Asp Asn Glu Glu Thr Phe Leu Lys
            100                 105                 110

Lys Tyr Leu Tyr Glu Ile Ala Arg Arg His Pro Tyr Phe Tyr Ala Pro
        115                 120                 125

Glu Leu Leu Phe Phe Ala Lys Arg Tyr Lys Ala Ala Phe Thr Glu Cys
    130                 135                 140

Cys Gln Ala Ala Asp Lys Ala Ala Cys Leu Leu Pro Lys Leu Asp Glu
145                 150                 155                 160

Leu Arg Asp Glu Gly Lys Ala Ser Ser Ala Lys Gln Arg Leu Lys Cys
                165                 170                 175

Ala Ser Leu Gln Lys Phe Gly Glu Arg Ala Phe Lys Ala Trp Ala Val
            180                 185                 190

Ala Arg Leu Ser Gln Arg Phe Pro Lys Ala Glu Phe Ala Glu Val Ser
        195                 200                 205

Lys Leu Val Thr Asp Leu Thr Lys Val His Thr Glu Cys Cys His Gly
    210                 215                 220

Asp Leu Leu Glu Cys Ala Asp Asp Arg Ala Asp Leu Ala Lys Tyr Ile
225                 230                 235                 240

Cys Glu Asn Gln Asp Ser Ile Ser Ser Lys Leu Lys Glu Cys Cys Glu
                245                 250                 255

Lys Pro Leu Leu Glu Lys Ser His Cys Ile Ala Glu Val Glu Asn Asp
            260                 265                 270

Glu Met Pro Ala Asp Leu Pro Ser Leu Ala Ala Asp Phe Val Glu Ser
        275                 280                 285

Lys Asp Val Cys Lys Asn Tyr Ala Glu Ala Lys Asp Val Phe Leu Gly
    290                 295                 300

Met Phe Leu Tyr Glu Tyr Ala Arg Arg His Pro Asp Tyr Ser Val Val
305                 310                 315                 320

Leu Leu Leu Arg Leu Ala Lys Thr Tyr Glu Thr Thr Leu Glu Lys Cys
                325                 330                 335

Cys Ala Ala Ala Asp Pro His Glu Cys Tyr Ala Lys Val Phe Asp Glu
            340                 345                 350

Phe Lys Pro Leu Val Glu Glu Pro Gln Asn Leu Ile Lys Gln Asn Cys
        355                 360                 365

Glu Leu Phe Glu Gln Leu Gly Glu Tyr Lys Phe Gln Asn Ala Leu Leu
    370                 375                 380

Val Arg Tyr Thr Lys Lys Val Pro Gln Val Ser Thr Pro Thr Leu Val
385                 390                 395                 400

Glu Val Ser Arg Asn Leu Gly Lys Val Gly Ser Lys Cys Cys Lys His
                405                 410                 415

Pro Glu Ala Lys Arg Met Pro Cys Ala Glu Asp Tyr Leu Ser Val Val
            420                 425                 430

Leu Asn Gln Leu Cys Val Leu His Glu Lys Thr Pro Val Ser Asp Arg
        435                 440                 445

Val Thr Lys Cys Cys Thr Glu Ser Leu Val Asn Arg Arg Pro Cys Phe
    450                 455                 460

Ser Ala Leu Glu Val Asp Glu Thr Tyr Val Pro Lys Glu Phe Asn Ala
465                 470                 475                 480

Glu Thr Phe Thr Phe His Ala Asp Ile Cys Thr Leu Ser Glu Lys Glu
                485                 490                 495

Arg Gln Ile Lys Lys Gln Thr Ala Leu Val Glu Leu Val Lys His Lys
            500                 505                 510

Pro Lys Ala Thr Lys Glu Gln Leu Lys Ala Val Met Asp Asp Phe Ala
        515                 520                 525

Ala Phe Val Glu Lys Cys Cys Lys Ala Asp Asp Lys Glu Thr Cys Phe
    530                 535                 540

Ala Glu Glu Gly Lys Lys Thr Cys Cys Cys Lys Ser Ser Cys Leu Arg
545                 550                 555                 560

Leu Ile Thr Ser His Leu Lys Ala Ser Gln Pro Thr Met Arg Ile Arg
                565                 570                 575

Glu Arg Lys

<210>  14
<211>  580
<212>  PRT
<213>  Homo sapiens

<400>  14

Leu Ile Ala Phe Ala Gln Tyr Leu Gln Gln Cys Pro Phe Glu Asp His
1               5                   10                  15

Val Lys Leu Val Asn Glu Val Thr Glu Phe Ala Lys Thr Cys Val Ala
            20                  25                  30

Asp Glu Ser Ala Glu Asn Cys Asp Lys Ser Leu His Thr Leu Phe Gly
        35                  40                  45

Asp Lys Leu Cys Thr Val Ala Thr Leu Arg Glu Thr Tyr Gly Glu Met
    50                  55                  60

Ala Asp Cys Cys Ala Lys Gln Glu Pro Glu Arg Asn Glu Cys Phe Leu
65                  70                  75                  80

Gln His Lys Asp Asp Asn Pro Asn Leu Pro Arg Leu Val Arg Pro Glu
                85                  90                  95

Val Asp Val Met Cys Thr Ala Phe His Asp Asn Glu Glu Thr Phe Leu
            100                 105                 110

Lys Lys Tyr Leu Tyr Glu Ile Ala Arg Arg His Pro Tyr Phe Tyr Ala
        115                 120                 125

Pro Glu Leu Leu Phe Phe Ala Lys Arg Tyr Lys Ala Ala Phe Thr Glu
    130                 135                 140

Cys Cys Gln Ala Ala Asp Lys Ala Ala Cys Leu Leu Pro Lys Leu Asp
145                 150                 155                 160

Glu Leu Arg Asp Glu Gly Lys Ala Ser Ser Ala Lys Gln Arg Leu Lys
                165                 170                 175

Cys Ala Ser Leu Gln Lys Phe Gly Glu Arg Ala Phe Lys Ala Trp Ala
            180                 185                 190

Val Ala Arg Leu Ser Gln Arg Phe Pro Lys Ala Glu Phe Ala Glu Val
        195                 200                 205

Ser Lys Leu Val Thr Asp Leu Thr Lys Val His Thr Glu Cys Cys His
    210                 215                 220

Gly Asp Leu Leu Glu Cys Ala Asp Asp Arg Ala Asp Leu Ala Lys Tyr
225                 230                 235                 240

Ile Cys Glu Asn Gln Asp Ser Ile Ser Ser Lys Leu Lys Glu Cys Cys
                245                 250                 255

Glu Lys Pro Leu Leu Glu Lys Ser His Cys Ile Ala Glu Val Glu Asn
            260                 265                 270

Asp Glu Met Pro Ala Asp Leu Pro Ser Leu Ala Ala Asp Phe Val Glu
        275                 280                 285

Ser Lys Asp Val Cys Lys Asn Tyr Ala Glu Ala Lys Asp Val Phe Leu
    290                 295                 300

Gly Met Phe Leu Tyr Glu Tyr Ala Arg Arg His Pro Asp Tyr Ser Val
305                 310                 315                 320

Val Leu Leu Leu Arg Leu Ala Lys Thr Tyr Glu Thr Thr Leu Glu Lys
                325                 330                 335

Cys Cys Ala Ala Ala Asp Pro His Glu Cys Tyr Ala Lys Val Phe Asp
            340                 345                 350

Glu Phe Lys Pro Leu Val Glu Glu Pro Gln Asn Leu Ile Lys Gln Asn
        355                 360                 365

Cys Glu Leu Phe Glu Gln Leu Gly Glu Tyr Lys Phe Gln Asn Ala Leu
    370                 375                 380

Leu Val Arg Tyr Thr Lys Lys Val Pro Gln Val Ser Thr Pro Thr Leu
385                 390                 395                 400

Val Glu Val Ser Arg Asn Leu Gly Lys Val Gly Ser Lys Cys Cys Lys
                405                 410                 415

His Pro Glu Ala Lys Arg Met Pro Cys Ala Glu Asp Tyr Leu Ser Val
            420                 425                 430

Val Leu Asn Gln Leu Cys Val Leu His Glu Lys Thr Pro Val Ser Asp
        435                 440                 445

Arg Val Thr Lys Cys Cys Thr Glu Ser Leu Val Asn Arg Arg Pro Cys
    450                 455                 460

Phe Ser Ala Leu Glu Val Asp Glu Thr Tyr Val Pro Lys Glu Phe Asn
465                 470                 475                 480

Ala Glu Thr Phe Thr Phe His Ala Asp Ile Cys Thr Leu Ser Glu Lys
                485                 490                 495

Glu Arg Gln Ile Lys Lys Gln Thr Ala Leu Val Glu Leu Val Lys His
            500                 505                 510

Lys Pro Lys Ala Thr Lys Glu Gln Leu Lys Ala Val Met Asp Asp Phe
        515                 520                 525

Ala Ala Phe Val Glu Lys Cys Cys Lys Ala Asp Asp Lys Glu Thr Cys
    530                 535                 540

Phe Ala Glu Glu Gly Lys Lys Thr Cys Cys Cys Lys Ser Ser Cys Leu
545                 550                 555                 560

Arg Leu Ile Thr Ser His Leu Lys Ala Ser Gln Pro Thr Met Arg Ile
                565                 570                 575

Arg Glu Arg Lys
            580

<210>  15
<211>  590
<212>  PRT
<213>  Homo sapiens

<400>  15

Leu Gly Glu Glu Asn Phe Lys Ala Leu Val Leu Ile Ala Phe Ala Gln
1               5                   10                  15

Tyr Leu Gln Gln Cys Pro Phe Glu Asp His Val Lys Leu Val Asn Glu
            20                  25                  30

Val Thr Glu Phe Ala Lys Thr Cys Val Ala Asp Glu Ser Ala Glu Asn
        35                  40                  45

Cys Asp Lys Ser Leu His Thr Leu Phe Gly Asp Lys Leu Cys Thr Val
    50                  55                  60

Ala Thr Leu Arg Glu Thr Tyr Gly Glu Met Ala Asp Cys Cys Ala Lys
65                  70                  75                  80

Gln Glu Pro Glu Arg Asn Glu Cys Phe Leu Gln His Lys Asp Asp Asn
                85                  90                  95

Pro Asn Leu Pro Arg Leu Val Arg Pro Glu Val Asp Val Met Cys Thr
            100                 105                 110

Ala Phe His Asp Asn Glu Glu Thr Phe Leu Lys Lys Tyr Leu Tyr Glu
        115                 120                 125

Ile Ala Arg Arg His Pro Tyr Phe Tyr Ala Pro Glu Leu Leu Phe Phe
    130                 135                 140

Ala Lys Arg Tyr Lys Ala Ala Phe Thr Glu Cys Cys Gln Ala Ala Asp
145                 150                 155                 160

Lys Ala Ala Cys Leu Leu Pro Lys Leu Asp Glu Leu Arg Asp Glu Gly
                165                 170                 175

Lys Ala Ser Ser Ala Lys Gln Arg Leu Lys Cys Ala Ser Leu Gln Lys
            180                 185                 190

Phe Gly Glu Arg Ala Phe Lys Ala Trp Ala Val Ala Arg Leu Ser Gln
        195                 200                 205

Arg Phe Pro Lys Ala Glu Phe Ala Glu Val Ser Lys Leu Val Thr Asp
    210                 215                 220

Leu Thr Lys Val His Thr Glu Cys Cys His Gly Asp Leu Leu Glu Cys
225                 230                 235                 240

Ala Asp Asp Arg Ala Asp Leu Ala Lys Tyr Ile Cys Glu Asn Gln Asp
                245                 250                 255

Ser Ile Ser Ser Lys Leu Lys Glu Cys Cys Glu Lys Pro Leu Leu Glu
            260                 265                 270

Lys Ser His Cys Ile Ala Glu Val Glu Asn Asp Glu Met Pro Ala Asp
        275                 280                 285

Leu Pro Ser Leu Ala Ala Asp Phe Val Glu Ser Lys Asp Val Cys Lys
    290                 295                 300

Asn Tyr Ala Glu Ala Lys Asp Val Phe Leu Gly Met Phe Leu Tyr Glu
305                 310                 315                 320

Tyr Ala Arg Arg His Pro Asp Tyr Ser Val Val Leu Leu Leu Arg Leu
                325                 330                 335

Ala Lys Thr Tyr Glu Thr Thr Leu Glu Lys Cys Cys Ala Ala Ala Asp
            340                 345                 350

Pro His Glu Cys Tyr Ala Lys Val Phe Asp Glu Phe Lys Pro Leu Val
        355                 360                 365

Glu Glu Pro Gln Asn Leu Ile Lys Gln Asn Cys Glu Leu Phe Glu Gln
    370                 375                 380

Leu Gly Glu Tyr Lys Phe Gln Asn Ala Leu Leu Val Arg Tyr Thr Lys
385                 390                 395                 400

Lys Val Pro Gln Val Ser Thr Pro Thr Leu Val Glu Val Ser Arg Asn
                405                 410                 415

Leu Gly Lys Val Gly Ser Lys Cys Cys Lys His Pro Glu Ala Lys Arg
            420                 425                 430

Met Pro Cys Ala Glu Asp Tyr Leu Ser Val Val Leu Asn Gln Leu Cys
        435                 440                 445

Val Leu His Glu Lys Thr Pro Val Ser Asp Arg Val Thr Lys Cys Cys
    450                 455                 460

Thr Glu Ser Leu Val Asn Arg Arg Pro Cys Phe Ser Ala Leu Glu Val
465                 470                 475                 480

Asp Glu Thr Tyr Val Pro Lys Glu Phe Asn Ala Glu Thr Phe Thr Phe
                485                 490                 495

His Ala Asp Ile Cys Thr Leu Ser Glu Lys Glu Arg Gln Ile Lys Lys
            500                 505                 510

Gln Thr Ala Leu Val Glu Leu Val Lys His Lys Pro Lys Ala Thr Lys
        515                 520                 525

Glu Gln Leu Lys Ala Val Met Asp Asp Phe Ala Ala Phe Val Glu Lys
    530                 535                 540

Cys Cys Lys Ala Asp Asp Lys Glu Thr Cys Phe Ala Glu Glu Gly Lys
545                 550                 555                 560

Lys Thr Cys Cys Cys Lys Ser Ser Cys Leu Arg Leu Ile Thr Ser His
                565                 570                 575

Leu Lys Ala Ser Gln Pro Thr Met Arg Ile Arg Glu Arg Lys
            580                 585                 590

<210> 16
<211> 603
<212> PRT
<213> Homo sapiens

<400> 16

Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Cys Pro Phe Glu Asp His Val Lys Leu Val Asn Glu Val Thr Glu
        35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145                 150                 155                 160

Tyr Lys Ala Ala Phe Thr Glu Cys Cys Gln Ala Ala Asp Lys Ala Ala
                165                 170                 175

Cys Leu Leu Pro Lys Leu Asp Glu Leu Arg Asp Glu Gly Lys Ala Ser
            180                 185                 190

Ser Ala Lys Gln Arg Leu Lys Cys Ala Ser Leu Gln Lys Phe Gly Glu
        195                 200                 205

Arg Ala Phe Lys Ala Trp Ala Val Ala Arg Leu Ser Gln Arg Phe Pro
    210                 215                 220

Lys Ala Glu Phe Ala Glu Val Ser Lys Leu Val Thr Asp Leu Thr Lys

225                 230                 235                 240

Val His Thr Glu Cys Cys His Gly Asp Leu Leu Glu Cys Ala Asp Asp
                245                 250                 255

Arg Ala Asp Leu Ala Lys Tyr Ile Cys Glu Asn Gln Asp Ser Ile Ser
            260                 265                 270

Ser Lys Leu Lys Glu Cys Cys Glu Lys Pro Leu Leu Glu Lys Ser His
        275                 280                 285

Cys Ile Ala Glu Val Glu Asn Asp Glu Met Pro Ala Asp Leu Pro Ser
    290                 295                 300

Leu Ala Ala Asp Phe Val Glu Ser Lys Asp Val Cys Lys Asn Tyr Ala
305                 310                 315                 320

Glu Ala Lys Asp Val Phe Leu Gly Met Phe Leu Tyr Glu Tyr Ala Arg
                325                 330                 335

Arg His Pro Asp Tyr Ser Val Val Leu Leu Leu Arg Leu Ala Lys Thr
            340                 345                 350

Tyr Glu Thr Thr Leu Glu Lys Cys Cys Ala Ala Ala Asp Pro His Glu
        355                 360                 365

Cys Tyr Ala Lys Val Phe Asp Glu Phe Lys Pro Leu Val Glu Glu Pro
    370                 375                 380

Gln Asn Leu Ile Lys Gln Asn Cys Glu Leu Phe Glu Gln Leu Gly Glu
385                 390                 395                 400

Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys
            420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Asn Ala Glu Thr Phe Thr Phe His Ala Asp

500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540

Lys Ala Val Met Asp Asp Phe Ala Ala Phe Val Glu Lys Cys Cys Lys
545                 550                 555                 560

Ala Asp Asp Lys Glu Thr Cys Phe Ala Glu Glu Gly Lys Lys Thr Cys
                565                 570                 575

Cys Cys Lys Ser Ser Cys Leu Arg Leu Ile Thr Ser His Leu Lys Ala
            580                 585                 590

Ser Gln Pro Thr Met Arg Ile Arg Glu Arg Lys
        595                 600

<210>  17
<211>  5
<212>  PRT
<213>  artificial

<220>
<223>  artificial

<220>
<221>  MISC_FEATURE
<222>  (5)..(5)
<223>  Xaa represent between 0 to 23 consecutive amino acid residues of
       the amino acid residues 5 to 27 as shown in Seq-ID No. 16

<400>  17

Asp Ala His Lys Xaa
1               5

<210>  18
<211>  153
<212>  PRT
<213>  homo sapiens

<400>  18

Met Val Ala Thr Lys Thr Phe Ala Leu Leu Leu Leu Ser Leu Phe Leu
1               5                   10                  15

Ala Val Gly Leu Gly Glu Lys Lys Glu Gly His Phe Ser Ala Leu Pro
            20                  25                  30

Ser Leu Pro Val Gly Ser His Ala Lys Val Ser Ser Pro Gln Pro Arg
        35                  40                  45

Gly Pro Arg Tyr Ala Glu Gly Thr Phe Ile Ser Asp Tyr Ser Ile Ala

50                  55                  60

Met Asp Lys Ile His Gln Gln Asp Phe Val Asn Trp Leu Leu Ala Gln
65                  70                  75                  80

Lys Gly Lys Lys Asn Asp Trp Lys His Asn Ile Thr Gln Arg Glu Ala
                85                  90                  95

Arg Ala Leu Glu Leu Ala Ser Gln Ala Asn Arg Lys Glu Glu Glu Ala
            100                 105                 110

Val Glu Pro Gln Ser Ser Pro Ala Lys Asn Pro Ser Asp Glu Asp Leu
        115                 120                 125

Leu Arg Asp Leu Leu Ile Gln Glu Leu Leu Ala Cys Leu Leu Asp Gln
    130                 135                 140

Thr Asn Leu Cys Arg Leu Arg Ser Arg
145                 150

<210>   19
<211>   30
<212>   PRT
<213>   homo sapiens

<400>   19

Glu Lys Lys Glu Gly His Phe Ser Ala Leu Pro Ser Leu Pro Val Gly
1               5                   10                  15

Ser His Ala Lys Val Ser Ser Pro Gln Pro Arg Gly Pro Arg
            20                  25                  30

<210>   20
<211>   22
<212>   PRT
<213>   homo sapiens

<400>   20

Ala Leu Pro Ser Leu Pro Val Gly Ser His Ala Lys Val Ser Ser Pro
1               5                   10                  15

Gln Pro Arg Gly Pro Arg
            20

<210>   21
<211>   60
<212>   PRT
<213>   homo sapiens

<400>   21

Arg Glu Ala Arg Ala Leu Glu Leu Ala Ser Gln Ala Asn Arg Lys Glu
1               5                   10                  15

Glu Glu Ala Val Glu Pro Gln Ser Ser Pro Ala Lys Asn Pro Ser Asp
            20                  25                  30

Glu Asp Leu Leu Arg Asp Leu Leu Ile Gln Glu Leu Leu Ala Cys Leu
        35                  40                  45

Leu Asp Gln Thr Asn Leu Cys Arg Leu Arg Ser Arg
    50                  55                  60

<210>  22
<211>  42
<212>  PRT
<213>  homo sapiens

<400>  22

Tyr Ala Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>  23
<211>  40
<212>  PRT
<213>  homo sapiens

<400>  23

Glu Gly Thr Phe Ile Ser Asp Tyr Ser Ile Ala Met Asp Lys Ile His
1               5                   10                  15

Gln Gln Asp Phe Val Asn Trp Leu Leu Ala Gln Lys Gly Lys Lys Asn
            20                  25                  30

Asp Trp Lys His Asn Ile Thr Gln
        35                  40